# EUROPEAN PATENT APPLICATION

(11) **EP 2 599 867 A1**
(43) Date of publication of application: **05.06.2013**
(21) Application number: 11812569.9
(22) Date of filing: 28.07.2011
(51) Int. Cl.: C12N 15/09, C12N 9/10

(54) **NOVEL ENZYME PROTEIN, PROCESS FOR PRODUCTION OF THE ENZYME PROTEIN, AND GENE ENCODING THE ENZYME PROTEIN**

(30) Priority: 30.07.2010 JP 2010171688
(71) Applicant: JAPAN TOBACCO INC., Minato-ku Tokyo 105-8422 (JP)
(72) Inventor: MINE, Toshiki, Iwata-shi Shizuoka 438-0802 (JP); YAMAMOTO, Takeshi, Yokohama-shi Kanagawa 227-8512 (JP); KATAYAMA, Sakurako, Iwata-shi Shizuoka 438-0802 (JP)
(74) Representative: Isarpatent
(86) International application number: PCT/JP2011/067265
(87) International publication number: WO 2012/014980

(57) **Abstract**

The present invention relates to a protein having a β-galactoside-α2,3-sialyltransferase activity but substantially not having any neuraminidase activity, a nucleic acid encoding the enzyme protein, and a method of producing the enzyme using a microorganism transformed with a gene encoding the protein. The protein of the present invention is a modified-type enzyme protein produced by mutating a specific amino acid residue in a β-galactoside-α2,3-sialyltransferase protein derived from a microorganism belonging to the genus Photobacterium of the family Vibrionaceae and thereby substantially inactivating the neuraminidase activity of the protein.

SEQUENCE LISTING

<110>  Japan Tobacco, Inc.
       Mine, Toshiki
       Takeshi, Yamamoto
       Sakurako, Katayama

<120>  A novel enzyme protein, a method for producing said enzyme protein,
and a gene encoding said enzyme protein

<130>  FA0392-11056

<150>  JP2010-171688
<160>  2010-07-30

<160>  26

<170>  PatentIn version 3.1

<210>  1
<211>  1161
<212>  DNA
<213>  Artificial sequence

<220>
<223>  beta-galactoside-alpha2,3-sialyltransferase N2C0

<220>
<221>  CDS
<222>  (1)..(1161)
<223>  /product="beta-galactoside-alpha2,3-sialyltransferase N2C0"

<400>  1
atg gac tct aag cac aat aac tca gat ggt aat att aca aaa aat aaa      48
Met Asp Ser Lys His Asn Asn Ser Asp Gly Asn Ile Thr Lys Asn Lys
1               5                   10                  15

aca ata gaa gtt tat gtt gat aga gca aca tta cca act att caa caa      96
Thr Ile Glu Val Tyr Val Asp Arg Ala Thr Leu Pro Thr Ile Gln Gln
            20                  25                  30

atg act cag att att aat gaa aat tca aat aat aag aaa ctt att tct      144
Met Thr Gln Ile Ile Asn Glu Asn Ser Asn Asn Lys Lys Leu Ile Ser
        35                  40                  45

tgg tct cga tac cct att aat gat gaa acg tta tta gaa tca att aat      192
Trp Ser Arg Tyr Pro Ile Asn Asp Glu Thr Leu Leu Glu Ser Ile Asn
    50                  55                  60

gga tca ttt ttt aaa aat agg cca gag cta att aaa tct ctt gat tct      240
Gly Ser Phe Phe Lys Asn Arg Pro Glu Leu Ile Lys Ser Leu Asp Ser
65                  70                  75                  80

atg ata ctt act aat gag att aaa aaa gta atc att aat ggt aat acc      288
Met Ile Leu Thr Asn Glu Ile Lys Lys Val Ile Ile Asn Gly Asn Thr
                85                  90                  95

tta tgg gca gta gat gtc gtt aat att ata aaa tca att gaa gct ctt      336
Leu Trp Ala Val Asp Val Val Asn Ile Ile Lys Ser Ile Glu Ala Leu
            100                 105                 110

gga aaa aaa aca gag att gaa cta aat ttt tat gat gac ggt agt gca      384

Gly Lys Lys Thr Glu Ile Glu Leu Asn Phe Tyr Asp Asp Gly Ser Ala
        115                 120                 125

gaa tat gtt cga tta tat gac ttt tca aga tta cct gaa tca gaa caa      432
Glu Tyr Val Arg Leu Tyr Asp Phe Ser Arg Leu Pro Glu Ser Glu Gln
    130                 135                 140

gaa tat aaa ata tcc tta tca aag gat aac att caa tca agt ata aat      480
Glu Tyr Lys Ile Ser Leu Ser Lys Asp Asn Ile Gln Ser Ser Ile Asn
145                 150                 155                 160

gga act caa cca ttt gat aac tca att gaa aat atc tat ggc ttt tcg      528
Gly Thr Gln Pro Phe Asp Asn Ser Ile Glu Asn Ile Tyr Gly Phe Ser
                165                 170                 175

cag tta tac cca aca aca tat cat atg ctc aga gca gat att ttt gaa      576
Gln Leu Tyr Pro Thr Thr Tyr His Met Leu Arg Ala Asp Ile Phe Glu
            180                 185                 190

act aat tta cct ttg acc tct ttg aaa aga gta ata tca aat aat att      624
Thr Asn Leu Pro Leu Thr Ser Leu Lys Arg Val Ile Ser Asn Asn Ile
        195                 200                 205

aag caa atg aaa tgg gat tat ttt aca act ttt aat tcc caa cag aag      672
Lys Gln Met Lys Trp Asp Tyr Phe Thr Thr Phe Asn Ser Gln Gln Lys
    210                 215                 220

aat aaa ttc tat aat ttc acg gga ttt aac cca gaa aaa att aag gaa      720
Asn Lys Phe Tyr Asn Phe Thr Gly Phe Asn Pro Glu Lys Ile Lys Glu
225                 230                 235                 240

caa tat aaa gca agc cct cat gaa aat ttt att ttt atc gga act aat      768
Gln Tyr Lys Ala Ser Pro His Glu Asn Phe Ile Phe Ile Gly Thr Asn
                245                 250                 255

tca gga aca gca acg gca gag caa caa ata gat att ctt aca gaa gct      816
Ser Gly Thr Ala Thr Ala Glu Gln Gln Ile Asp Ile Leu Thr Glu Ala
            260                 265                 270

aaa aag cca gat agc ccg ata ata act aat tca att caa gga ttg gat      864
Lys Lys Pro Asp Ser Pro Ile Ile Thr Asn Ser Ile Gln Gly Leu Asp
        275                 280                 285

ttg ttt ttc aaa gga cat ccg agt gca act tat aat caa caa atc att      912
Leu Phe Phe Lys Gly His Pro Ser Ala Thr Tyr Asn Gln Gln Ile Ile
    290                 295                 300

gat gct cat aat atg att gaa att tat aat aag ata cca ttt gaa gct      960
Asp Ala His Asn Met Ile Glu Ile Tyr Asn Lys Ile Pro Phe Glu Ala
305                 310                 315                 320

cta ata atg act gat gca ttg cct gat gct gtc ggt gga atg gga agt     1008
Leu Ile Met Thr Asp Ala Leu Pro Asp Ala Val Gly Gly Met Gly Ser
                325                 330                 335

tcg gta ttt ttt agc ttg cca aat aca gta gag aat aaa ttt att ttt     1056
Ser Val Phe Phe Ser Leu Pro Asn Thr Val Glu Asn Lys Phe Ile Phe
            340                 345                 350

tat aaa agt gat acg gat att gaa aat aat gct ctt ata caa gta atg     1104
Tyr Lys Ser Asp Thr Asp Ile Glu Asn Asn Ala Leu Ile Gln Val Met
        355                 360                 365

att gaa ctg aat atc gtt aat aga aat gat gtt aag ttg ata agt gat     1152
Ile Glu Leu Asn Ile Val Asn Arg Asn Asp Val Lys Leu Ile Ser Asp
    370                 375                 380

ttg cag taa                                                         1161
Leu Gln
385

<210>  2
<211>  386
<212>  PRT
<213>  Artificial sequence

<220>
<223>  beta-galactoside-alpha2,3-sialyltransferase N2C0

<400>  2

Met Asp Ser Lys His Asn Asn Ser Asp Gly Asn Ile Thr Lys Asn Lys
1               5                   10                  15

Thr Ile Glu Val Tyr Val Asp Arg Ala Thr Leu Pro Thr Ile Gln Gln
            20                  25                  30

Met Thr Gln Ile Ile Asn Glu Asn Ser Asn Asn Lys Lys Leu Ile Ser
        35                  40                  45

Trp Ser Arg Tyr Pro Ile Asn Asp Glu Thr Leu Leu Glu Ser Ile Asn
    50                  55                  60

Gly Ser Phe Phe Lys Asn Arg Pro Glu Leu Ile Lys Ser Leu Asp Ser
65                  70                  75                  80

Met Ile Leu Thr Asn Glu Ile Lys Lys Val Ile Ile Asn Gly Asn Thr
                85                  90                  95

Leu Trp Ala Val Asp Val Val Asn Ile Ile Lys Ser Ile Glu Ala Leu
            100                 105                 110

Gly Lys Lys Thr Glu Ile Glu Leu Asn Phe Tyr Asp Asp Gly Ser Ala
        115                 120                 125

Glu Tyr Val Arg Leu Tyr Asp Phe Ser Arg Leu Pro Glu Ser Glu Gln
    130                 135                 140

Glu Tyr Lys Ile Ser Leu Ser Lys Asp Asn Ile Gln Ser Ser Ile Asn
145                 150                 155                 160

Gly Thr Gln Pro Phe Asp Asn Ser Ile Glu Asn Ile Tyr Gly Phe Ser
                165                 170                 175

Gln Leu Tyr Pro Thr Thr Tyr His Met Leu Arg Ala Asp Ile Phe Glu
            180                 185                 190

Thr Asn Leu Pro Leu Thr Ser Leu Lys Arg Val Ile Ser Asn Asn Ile
        195                 200                 205

Lys Gln Met Lys Trp Asp Tyr Phe Thr Thr Phe Asn Ser Gln Gln Lys
    210                 215                 220

Asn Lys Phe Tyr Asn Phe Thr Gly Phe Asn Pro Glu Lys Ile Lys Glu
225                 230                 235                 240

Gln Tyr Lys Ala Ser Pro His Glu Asn Phe Ile Phe Ile Gly Thr Asn
                245                 250                 255

Ser Gly Thr Ala Thr Ala Glu Gln Gln Ile Asp Ile Leu Thr Glu Ala
            260                 265                 270

Lys Lys Pro Asp Ser Pro Ile Ile Thr Asn Ser Ile Gln Gly Leu Asp
        275                 280                 285

Leu Phe Phe Lys Gly His Pro Ser Ala Thr Tyr Asn Gln Gln Ile Ile
    290                 295                 300

Asp Ala His Asn Met Ile Glu Ile Tyr Asn Lys Ile Pro Phe Glu Ala
305                 310                 315                 320

Leu Ile Met Thr Asp Ala Leu Pro Asp Ala Val Gly Gly Met Gly Ser
                325                 330                 335

Ser Val Phe Phe Ser Leu Pro Asn Thr Val Glu Asn Lys Phe Ile Phe
            340                 345                 350

Tyr Lys Ser Asp Thr Asp Ile Glu Asn Asn Ala Leu Ile Gln Val Met
        355                 360                 365

Ile Glu Leu Asn Ile Val Asn Arg Asn Asp Val Lys Leu Ile Ser Asp
    370                 375                 380

Leu Gln
385

<210>  3
<211>  1230
<212>  DNA
<213>  Photobacterium phosphoreum

<220>
<221>  CDS

<222>   (1)..(1230)
<223>   /product="beta-galactoside-alpha2,3-sialyltransferase"

<400>   3
atg ttc gtt ttt tgt aaa aaa ata ttt ttt ttg att ttt att tca cta        48
Met Phe Val Phe Cys Lys Lys Ile Phe Phe Leu Ile Phe Ile Ser Leu
1               5                   10                  15

atg att ctg ggg ggc tgt aat agt gac tct aag cac aat aac tca gat        96
Met Ile Leu Gly Gly Cys Asn Ser Asp Ser Lys His Asn Asn Ser Asp
            20                  25                  30

ggt aat att aca aaa aat aaa aca ata gaa gtt tat gtt gat aga gca       144
Gly Asn Ile Thr Lys Asn Lys Thr Ile Glu Val Tyr Val Asp Arg Ala
        35                  40                  45

aca tta cca act att caa caa atg act cag att att aat gaa aat tca       192
Thr Leu Pro Thr Ile Gln Gln Met Thr Gln Ile Ile Asn Glu Asn Ser
    50                  55                  60

aat aat aag aaa ctt att tct tgg tct cga tac cct att aat gat gaa       240
Asn Asn Lys Lys Leu Ile Ser Trp Ser Arg Tyr Pro Ile Asn Asp Glu
65                  70                  75                  80

acg tta tta gaa tca att aat gga tca ttt ttt aaa aat agg cca gag       288
Thr Leu Leu Glu Ser Ile Asn Gly Ser Phe Phe Lys Asn Arg Pro Glu
                85                  90                  95

cta att aaa tct ctt gat tct atg ata ctt act aat gag att aaa aaa       336
Leu Ile Lys Ser Leu Asp Ser Met Ile Leu Thr Asn Glu Ile Lys Lys
            100                 105                 110

gta atc att aat ggt aat acc tta tgg gca gta gat gtc gtt aat att       384
Val Ile Ile Asn Gly Asn Thr Leu Trp Ala Val Asp Val Val Asn Ile
        115                 120                 125

ata aaa tca att gaa gct ctt gga aaa aaa aca gag att gaa cta aat       432
Ile Lys Ser Ile Glu Ala Leu Gly Lys Lys Thr Glu Ile Glu Leu Asn
    130                 135                 140

ttt tat gat gac ggt agt gca gaa tat gtt cga tta tat gac ttt tca       480
Phe Tyr Asp Asp Gly Ser Ala Glu Tyr Val Arg Leu Tyr Asp Phe Ser
145                 150                 155                 160

aga tta cct gaa tca gaa caa gaa tat aaa ata tcc tta tca aag gat       528
Arg Leu Pro Glu Ser Glu Gln Glu Tyr Lys Ile Ser Leu Ser Lys Asp
                165                 170                 175

aac att caa tca agt ata aat gga act caa cca ttt gat aac tca att       576
Asn Ile Gln Ser Ser Ile Asn Gly Thr Gln Pro Phe Asp Asn Ser Ile
            180                 185                 190

gaa aat atc tat ggc ttt tcg cag tta tac cca aca aca tat cat atg       624
Glu Asn Ile Tyr Gly Phe Ser Gln Leu Tyr Pro Thr Thr Tyr His Met
        195                 200                 205

ctc aga gca gat att ttt gaa act aat tta cct ttg acc tct ttg aaa       672
Leu Arg Ala Asp Ile Phe Glu Thr Asn Leu Pro Leu Thr Ser Leu Lys
    210                 215                 220

aga gta ata tca aat aat att aag caa atg aaa tgg gat tat ttt aca       720
Arg Val Ile Ser Asn Asn Ile Lys Gln Met Lys Trp Asp Tyr Phe Thr

225                 230                 235                 240

act ttt aat tcc caa cag aag aat aaa ttc tat aat ttc acg gga ttt       768
Thr Phe Asn Ser Gln Gln Lys Asn Lys Phe Tyr Asn Phe Thr Gly Phe
                245                 250                 255

aac cca gaa aaa att aag gaa caa tat aaa gca agc cct cat gaa aat       816
Asn Pro Glu Lys Ile Lys Glu Gln Tyr Lys Ala Ser Pro His Glu Asn
            260                 265                 270

ttt att ttt atc gga act aat tca gga aca gca acg gca gag caa caa       864
Phe Ile Phe Ile Gly Thr Asn Ser Gly Thr Ala Thr Ala Glu Gln Gln
        275                 280                 285

ata gat att ctt aca gaa gct aaa aag cca gat agc ccg ata ata act       912
Ile Asp Ile Leu Thr Glu Ala Lys Lys Pro Asp Ser Pro Ile Ile Thr
    290                 295                 300

aat tca att caa gga ttg gat ttg ttt ttc aaa gga cat ccg agt gca       960
Asn Ser Ile Gln Gly Leu Asp Leu Phe Phe Lys Gly His Pro Ser Ala
305                 310                 315                 320

act tat aat caa caa atc att gat gct cat aat atg att gaa att tat      1008
Thr Tyr Asn Gln Gln Ile Ile Asp Ala His Asn Met Ile Glu Ile Tyr
                325                 330                 335

aat aag ata cca ttt gaa gct cta ata atg act gat gca ttg cct gat      1056
Asn Lys Ile Pro Phe Glu Ala Leu Ile Met Thr Asp Ala Leu Pro Asp
            340                 345                 350

gct gtc ggt gga atg gga agt tcg gta ttt ttt agc ttg cca aat aca      1104
Ala Val Gly Gly Met Gly Ser Ser Val Phe Phe Ser Leu Pro Asn Thr
        355                 360                 365

gta gag aat aaa ttt att ttt tat aaa agt gat acg gat att gaa aat      1152
Val Glu Asn Lys Phe Ile Phe Tyr Lys Ser Asp Thr Asp Ile Glu Asn
    370                 375                 380

aat gct ctt ata caa gta atg att gaa ctg aat atc gtt aat aga aat      1200
Asn Ala Leu Ile Gln Val Met Ile Glu Leu Asn Ile Val Asn Arg Asn
385                 390                 395                 400

gat gtt aag ttg ata agt gat ttg cag taa                              1230
Asp Val Lys Leu Ile Ser Asp Leu Gln
                405

<210>  4
<211>  409
<212>  PRT
<213>  Photobacterium phosphoreum

<400>  4

Met Phe Val Phe Cys Lys Lys Ile Phe Phe Leu Ile Phe Ile Ser Leu
1               5                   10                  15

Met Ile Leu Gly Gly Cys Asn Ser Asp Ser Lys His Asn Asn Ser Asp
            20                  25                  30

Gly Asn Ile Thr Lys Asn Lys Thr Ile Glu Val Tyr Val Asp Arg Ala

35                  40                  45

Thr Leu Pro Thr Ile Gln Gln Met Thr Gln Ile Ile Asn Glu Asn Ser
    50                  55                  60

Asn Asn Lys Lys Leu Ile Ser Trp Ser Arg Tyr Pro Ile Asn Asp Glu
65                  70                  75                  80

Thr Leu Leu Glu Ser Ile Asn Gly Ser Phe Phe Lys Asn Arg Pro Glu
                85                  90                  95

Leu Ile Lys Ser Leu Asp Ser Met Ile Leu Thr Asn Glu Ile Lys Lys
            100                 105                 110

Val Ile Ile Asn Gly Asn Thr Leu Trp Ala Val Asp Val Val Asn Ile
        115                 120                 125

Ile Lys Ser Ile Glu Ala Leu Gly Lys Lys Thr Glu Ile Glu Leu Asn
    130                 135                 140

Phe Tyr Asp Asp Gly Ser Ala Glu Tyr Val Arg Leu Tyr Asp Phe Ser
145                 150                 155                 160

Arg Leu Pro Glu Ser Glu Gln Glu Tyr Lys Ile Ser Leu Ser Lys Asp
                165                 170                 175

Asn Ile Gln Ser Ser Ile Asn Gly Thr Gln Pro Phe Asp Asn Ser Ile
            180                 185                 190

Glu Asn Ile Tyr Gly Phe Ser Gln Leu Tyr Pro Thr Thr Tyr His Met
        195                 200                 205

Leu Arg Ala Asp Ile Phe Glu Thr Asn Leu Pro Leu Thr Ser Leu Lys
    210                 215                 220

Arg Val Ile Ser Asn Asn Ile Lys Gln Met Lys Trp Asp Tyr Phe Thr
225                 230                 235                 240

Thr Phe Asn Ser Gln Gln Lys Asn Lys Phe Tyr Asn Phe Thr Gly Phe
                245                 250                 255

Asn Pro Glu Lys Ile Lys Glu Gln Tyr Lys Ala Ser Pro His Glu Asn
            260                 265                 270

Phe Ile Phe Ile Gly Thr Asn Ser Gly Thr Ala Thr Ala Glu Gln Gln
        275                 280                 285

Ile Asp Ile Leu Thr Glu Ala Lys Lys Pro Asp Ser Pro Ile Ile Thr
    290                 295                 300

Asn Ser Ile Gln Gly Leu Asp Leu Phe Phe Lys Gly His Pro Ser Ala
305                 310                 315                 320

Thr Tyr Asn Gln Gln Ile Ile Asp Ala His Asn Met Ile Glu Ile Tyr
                325                 330                 335

Asn Lys Ile Pro Phe Glu Ala Leu Ile Met Thr Asp Ala Leu Pro Asp
            340                 345                 350

Ala Val Gly Gly Met Gly Ser Ser Val Phe Phe Ser Leu Pro Asn Thr
        355                 360                 365

Val Glu Asn Lys Phe Ile Phe Tyr Lys Ser Asp Thr Asp Ile Glu Asn
    370                 375                 380

Asn Ala Leu Ile Gln Val Met Ile Glu Leu Asn Ile Val Asn Arg Asn
385                 390                 395                 400

Asp Val Lys Leu Ile Ser Asp Leu Gln
                405

<210>  5
<211>  8
<212>  PRT
<213>  Artificial sequence

<220>
<223>  FLAG tag

<400>  5

Asp Tyr Lys Asp Asp Asp Asp Lys
1               5

<210>  6
<211>  35
<212>  DNA
<213>  Artificial sequence

<220>
<223>  Primer ISH467-23ST-N2-Nco

<400>  6
gggctgtacc atggactcta agcacaataa ctcag                                35

<210>  7
<211>  36
<212>  DNA
<213>  Artificial sequence

<220>
<223>  Primer ISH467-23ST-C0-BamHI

<400>  7
cttagaatgg atccttactg caaatcactt atcaac                                36


<210>  8
<211>  61
<212>  DNA
<213>  Artificial sequence

<220>
<223>  Primer ISH467-23ST-C0-His-BamHI

<400>  8
ggggggatcc ttagtgatgg tgatggtgat gctgcaaatc acttatcaac ttaacatcat      60

t                                                                      61


<210>  9
<211>  46
<212>  DNA
<213>  Artificial sequence

<220>
<223>  Primer ISH467-23ST-F318A-F

<400>  9
aaatttataa taagatacca gctgaagctc taataatgac tgatgc                    46


<210>  10
<211>  46
<212>  DNA
<213>  Artificial sequence

<220>
<223>  Primer ISH467-23ST-F318A-R

<400>  10
gcatcagtca ttattagagc ttcagctggt atcttattat aaattt                    46


<210>  11
<211>  46
<212>  DNA
<213>  Artificial sequence

<220>
<223>  Primer ISH467-23ST-E319A-F

<400>  11
aaatttataa taagatacca tttgcagctc taataatgac tgatgc                    46


<210>  12
<211>  46
<212>  DNA
<213>  Artificial sequence

<220>

<223>  Primer ISH467-23ST-E319A-R

<400>  12
gcatcagtca ttattagagc tgcaaatggt atcttattat aaattt                    46


<210>  13
<211>  46
<212>  DNA
<213>  Artificial sequence

<220>
<223>  Primer ISH467-23ST-F318AE319A-F

<400>  13
aaatttataa taagatacca gctgcagctc taataatgac tgatgc                    46


<210>  14
<211>  46
<212>  DNA
<213>  Artificial sequence

<220>
<223>  Primer ISH467-23ST-F318AE319A-R

<400>  14
gcatcagtca ttattagagc tgcagctggt atcttattat aaattt                    46


<210>  15
<211>  43
<212>  DNA
<213>  Artificial sequence

<220>
<223>  Primer ISH467-23ST-S337A-F

<400>  15
ctgtcggtgg aatgggaagt gctgtatttt ttagcttgcc aaa                       43


<210>  16
<211>  43
<212>  DNA
<213>  Artificial sequence

<220>
<223>  Primer ISH467-23ST-S337A-R

<400>  16
tttggcaagc taaaaaatac agcacttccc attccaccga cag                       43


<210>  17
<211>  46
<212>  DNA
<213>  Artificial sequence

<220>
<223>  Primer ISH467-23ST-E319V-F

<400>  17

aaatttataa taagatacca tttgtggctc taataatgac tgatgc                46

<210>  18
<211>  46
<212>  DNA
<213>  Artificial sequence

<220>
<223>  Primer ISH467-23ST-E319V-R

<400>  18
gcatcagtca ttattagagc cacaaatggt atcttattat aaattt                46

<210>  19
<211>  46
<212>  DNA
<213>  Artificial sequence

<220>
<223>  Primer ISH467-23ST-E319S-F

<400>  19
aaatttataa taagatacca tttagcgctc taataatgac tgatgc                46

<210>  20
<211>  46
<212>  DNA
<213>  Artificial sequence

<220>
<223>  Primer ISH467-23ST-E319S-R

<400>  20
gcatcagtca ttattagagc gctaaatggt atcttattat aaattt                46

<210>  21
<211>  46
<212>  DNA
<213>  Artificial sequence

<220>
<223>  Primer ISH467-23ST-E319K-F

<400>  21
aaatttataa taagatacca tttaaagctc taataatgac tgatgc                46

<210>  22
<211>  46
<212>  DNA
<213>  Artificial sequence

<220>
<223>  Primer ISH467-23ST-E319K-R

<400>  22
gcatcagtca ttattagagc tttaaatggt atcttattat aaattt                46

<210>  23
<211>  46
<212>  DNA
<213>  Artificial sequence

<220>
<223>  Primer ISH467-23STE319H-F

<400>  23
aaatttataa taagatacca tttcatgctc taataatgac tgatgc                    46


<210>  24
<211>  46
<212>  DNA
<213>  Artificial sequence

<220>
<223>  Primer ISH467-23ST-E319H-R

<400>  24
gcatcagtca ttattagagc atgaaatggt atcttattat aaattt                    46


<210>  25
<211>  19
<212>  DNA
<213>  Artificial sequence

<220>
<223>  Primer pTrc99A FW5

<400>  25
ggaagctgtg gtatggctg                                                  19


<210>  26
<211>  20
<212>  DNA
<213>  Artificial sequence

<220>
<223>  Primer pTrc99A RV3

<400>  26
cgtttcactt ctgagttcgg                                                 20

## Description

### TECHNICAL FIELD

The present invention relates to a protein having a β-galactoside-α2,3-sialyltransferase activity but substantially not having any neuraminidase activity, a nucleic acid encoding the enzyme protein, and a method for producing the enzyme using a microorganism transformed with the gene encoding the protein.

### BACKGROUND ART

Sugar chains are compounds composed of various sugars, for example, monosaccharides such as galactose and N-acetylglucosamine. The sugar chains of, for example, glycoproteins or glycolipids (hereinafter referred to as complex carbohydrate chains) have very important functions *in vivo.* The past studies suggest that sugar chains containing monosaccharide named sialic acid, among the sugar chains, express particularly important functions. For example, it has been shown mainly in mammalian cells that sugar chains containing sialic acid are important molecules functioning in differentiation and growth for signaling between cells and between a cell and an extracellular matrix or as tags of complex carbohydrates and that complex carbohydrates containing sialic acid are prominently involved in the formation of synapses in brain and nerve cells, neurological development, and improvements in learning ability. Sialic acid is a general term for acyl derivatives of neuraminic acid and is one of the acidic monosaccharides having a carboxyl group in its structure. Until now, 50 or more molecular species have been identified in mainly mammals, echinoderms, and bacteria. Typical examples of the sialic acid that has been known include *N*-acetylneuraminic acid (Neu5Ac), *N*-glyeolylneuraminic acid (Neu5Gc), and deaminoneuraminic acid (KDN). Among these sialic acids, only *N-*acetylneuraminic acid is generally found in human body. It is known that *N-*glycolylneuraminic acid is present in some cancer cells, as a particular example.

Sialic acids, which are found in complex carbohydrate chains *in vivo,* such as glycoproteins and glycolipids, are transferred to sugar chains serving as sugar acceptor substrates by a group of glycotransferases called sialyltransferases. More specifically, sialyltransferases transfer sialic acid from a CMP-sialic acid, which is a common sugar donor substrate, to various sugar acceptor substrates.

Incidentally, neuraminidase (sialidase), which is an enzyme catalyzing a reaction of releasing a sialic acid residue located at a nonreducing terminus of a complex carbohydrate chain, is present *in vivo.* Until now, many neuraminidase proteins have been found in, for example, animals, microorganisms, and viruses, and genes encoding neuraminidase proteins have been cloned. Known main linkage modes of sialic acid are three types: α2,3-linkage, α2,6-linkage, and α2,8-linkage. Though a small number of types of neuraminidases semiselectively cleave sialic acid of which linkage mode is α2,3-linkage or α2,6-linkage, most neuraminidases that have been reported until now cleave sialic acid from a complex carbohydrate chain containing sialic acid regardless of the linkage mode of the sialic acid.

As described above, the complex carbohydrate chain containing sialic acid present in the body has very important functions *in vivo.* Accordingly, the above-mentioned sialyltransferases are one type of enzymes having high demand, for example, for detailed functional investigation of glycoproteins and glycolipids containing sialic acid or preparation of physiologically active substance using the functions.

The sialyltransferases that have been reported until now are classified into several groups, and there are many reports on those derived from animals, in particular, mammals (Hamamoto, T., et al., Bioorg. Med. Chem., 1, 141-145 (1993); and Weinstein, J., et al., J. Biol. Chem., 262, 17735-17743 (1987)). These animal-derived enzymes have a significantly high sugar acceptor substrate specificity and it can transfer sialic acid to limited types of sugar chain structures *in vivo.* That is, significantly limited glycoproteins and glycolipids can be generated by reactions of the sialyltransferases. On the other hand, sialyltransferases and their genes isolated from a microorganism belonging to *Photobacterium damselae* have been reported (International Publication No. WO 98/38315; United States Patent No. 6255094; Yamamoto, T., et al., J. Biochem., 120, 104-110 (1996); Tsukamoto, H., et al., J. Biol. Chem., 282, 29794-29802 (2007); and Takakura, Y., et al., J. Biochem., (Tokyo), 142, 403-412 (2007)). These sialyltransferases derived from microorganisms have a significantly broad sugar acceptor substrate specificity compared to the above-mentioned animal-derived enzymes, and it can transfer sialic acid to sugar chains to which the animal-derived sialyltransferases cannot transfer sialic acid. Accordingly, sialyltransferases derived from marine microorganisms, in particular, sialyltransferases derived from marine bacteria are extremely highly demanded enzymes, among these highly demanded sialyltransferases.

However, many marine bacteria-derived sialyltransferases that have been found until now are enzyme proteins having both a sialyltransferase activity and a neuraminidase activity (Tsukamoto, H., et al., J. Biol. Chem., 282, 29794-29802 (2007); and Takakura, Y., et al., J. Biochem., (Tokyo), 142, 403-412 (2007)). The inventors have found strains producing β-galactoside-α2,3-sialyltransferase activities, such as *Photobacterium sp.* strain JT-ISH-224 and *Photobacterium phosphoreum* strain JT-ISH-467, from strains of microorganisms belonging to the genus Photobacterium of the family Vibrionaceae or the genus Vibrio of the family Vibrionaceae (International Publication No. WO 2006/112253) and have demonstrated that these enzymes have both a sialyltransferase activity and a neuraminidase activity (Tsukamoto, H., et al., J. Biol. Chem., 282, 29794-29802 (2007)). In the case of using an enzyme having both the sialyltransferase activity and the neuraminidase activity, a long-term enzyme reaction causes the concentration of a sialic acid-containing compound as the reaction product to increase in the reaction system. The sialic acid-containing compound will then become a substrate of neuraminidase, and there is a risk that the sialic acid will be hydrolyzed from the reaction product. Accordingly, there are demands for a sialyltransferase having a reduced neuraminidase activity while maintaining the advantages of an enzyme derived from marine bacteria and for a gene thereof.

### CITATION LIST

### PATENT LITERATURE

Patent Literature 1: International Publication No. WO 98/38315
Patent Literature 2: U.S. Patent No. 6255094
Patent Literature 3: International Publication No. WO 2006/112253

### NON-PATENT LITERATURE

Non-Patent Literature 1: Hamamoto, T. , et al., Bioorg. Med. Chem., 1, 141-145 (1993)
Non-Patent Literature 2: Weinstein, J. , et al., J. Biol. Chem., 262, 17735-17743 (1987)
Non-Patent Literature 3: Yamamoto, T., et al., J. Biochem., 120, 104-110 (1996)
Non-Patent Literature 4: Tsukamoto, H., et al., J. Biol. Chem., 282, 29794-29802 (2007)
Non-Patent Literature 5: Takakura, Y., et al., J. Biochem. (Tokyo), 142, 403-412 (2007)

### SUMMARY OF INVENTION

### TECHNICAL PROBLEM

It is an object of the present invention to inactivate or considerably decrease the neuraminidase activity of an enzyme protein having a β-galactoside-α2,3-sialyltransferase activity derived from a microorganism belonging to the genus Photobacterium of the family Vibrionaceae. It is another object of the present invention to provide an enzyme protein having a β-galactoside-α2,3-sialyltransferase activity derived from a microorganism belonging to the genus Photobacterium of the family Vibrionaceae but substantially not having any neuraminidase activity, and to provide a gene encoding the enzyme protein.

### SOLUTION TO PROBLEM

The present inventors have found a strain (strain JT-ISH-467) producing a β-galactoside-α2,3-sialyltransferase activity from a microorganism, *Photobacterium phosphoreum,* belonging to the genus Photobacterium of the family Vibrionaceae and have revealed various properties of the enzyme (WO 2006/112253). The inventors have demonstrated that the enzyme has both a sialyltransferase activity and a neuraminidase activity (Non-Patent Literature 4).

Furthermore, the inventors have diligently studied and have found that a modified enzyme protein having a variation of glutamic acid at position 342 (corresponding to the glutamic acid at position 319 in SEQ ID NO: 2) of a β-galactoside-α2,3-sialyltransferase (SEQ ID NO: 4) derived from the *Photobacterium phosphoreum* strain JT-ISH-467 shows a β-galactoside-α2,3-sialyltransferase activity, but does not show a neuraminidase activity. The present invention provides a novel β-galactoside-α2,3-sialyltransferase protein substantially not having any neuraminidase activity, a nucleic acid encoding the protein, and a method of producing the protein.

Accordingly, the present invention provides the following embodiments:

Embodiment 1: A β-galactoside-α2,3-sialyltransferase protein comprising an amino acid sequence selected from the group consisting of:
(a) an amino acid sequence composed of 2nd to 386th amino acids of SEQ ID NO: 2 having replacement of the glutamic acid residue at position 319 of SEQ ID NO: 2 with an amino acid other than glutamic acid;
(b) an amino acid sequence having deletion, substitution, insertion, and/or addition of one or more amino acids in an amino acid sequence composed of 2nd to 386th amino acids of SEQ ID NO: 2, wherein at least the amino acid residue corresponding to the glutamic acid residue at position 319 of SEQ ID NO: 2 is replaced with an amino acid other than glutamic acid;
(c) an amino acid sequence having an identity of 80% or more with 2nd to 386th amino acids of SEQ ID NO: 2, wherein at least the amino acid residue corresponding to the glutamic acid residue at position 319 of SEQ ID NO: 2 is replaced with an amino acid other than glutamic acid; and
(d) an amino acid sequence encoded by a nucleotide sequence hybridizable with the complementary strand of 4th to 1158th nucleotides of SEQ ID NO: 1 under stringent conditions, wherein at least the codon corresponding to the 955th to the 957th nucleotides of SEQ ID NO: 1 is converted to a codon encoding an amino acid other than glutamic acid;
And wherein said protein substantially does not have any neuraminidase activity.

Embodiment 2: The protein according to Embodiment 1, wherein the amino acid sequence further includes methionine, the 1st to 24th amino acids of SEQ ID NO: 4, or a signal peptide at the N-terminus of the amino acid sequence specified by any one of items (a) to (d) in Embodiment 1.

Embodiment 3: The protein according to Embodiment 1 or 2, wherein the glutamic acid residue at position 319 of SEQ ID NO: 2 or the amino acid residue corresponding to the glutamic acid residue is replaced with a neutral amino acid residue or a basic amino acid residue.

Embodiment 4: The protein according to Embodiment 1 or 2, wherein the glutamic acid residue at position 319 of SEQ ID NO: 2 or the amino acid residue corresponding to the glutamic acid residue is replaced with a neutral amino acid residue.

Embodiment 5: The protein according to any one of Embodiments 1 to 4, satisfying one or more requirements selected from the group consisting of the following items a) to 1):
a) the tyrosine residue at position 123 of SEQ ID NO: 2 or at a position corresponding thereto, is not modified or is replaced with tryptophan or phenylalanine;
b) the aspartic acid residue at position 124 of SEQ ID NO: 2 or at a position corresponding thereto, is not modified or is replaced with glutamic acid;
c) the aspartic acid residue at position 125 of SEQ ID NO: 2 or at a position corresponding thereto, is not modified or is replaced with glutamic acid;
d) the glycine residue at position 126 of SEQ ID NO: 2 or at a position corresponding thereto, is not modified or is replaced with alanine or valine;
e) the serine residue at position 127 of SEQ ID NO: 2 or at a position corresponding thereto, is not modified or is replaced with threonine;
f) the phenylalanine residue at position 291 of SEQ ID NO: 2 or at a position corresponding thereto, is not modified or is replaced with tryptophan or tyrosine;
g) the lysine residue at position 292 of SEQ ID NO: 2 or at a position corresponding thereto, is not modified or is replaced with arginine;
h) the glycine residue at position 293 of SEQ ID NO: 2 or at a position corresponding thereto, is not modified or is replaced with alanine or valine;
i) the histidine residue at position 294 of SEQ ID NO: 2 or at a position corresponding thereto, is not modified or is replaced with lysine or arginine;
j) the proline residue at position 295 of SEQ ID NO: 2 or at a position corresponding thereto, is not modified or is replaced with valine or isoleucine;
k) the serine residue at position 336 of SEQ ID NO: 2 or at a position corresponding thereto, is not modified or is replaced with threonine; and
l) the serine residue at position 337 of SEQ ID NO: 2 or at a position corresponding thereto, is not modified or is replaced with threonine.

Embodiment 6: A nucleic acid encoding a protein according to any one of Embodiments 1 to 5.

Embodiment 7: An isolated nucleic acid encoding a β-galactoside-α2,3-sialyltransferase protein, wherein said protein is a β-galactoside-α2,3-sialyltransferase protein substantially not having any neuraminidase activity, and the nucleic acid comprising a nucleotide sequence selected from the group consisting of:
(a) a nucleotide sequence composed of the 4th to 1158th nucleotides of SEQ ID NO: 1 having a codon encoding an amino acid other than glutamic acid in place of the codon of the 955th to the 957th nucleotides of SEQ ID NO: 1;
(b) a nucleotide sequence having deletion, substitution, insertion, and/or addition of one or more nucleotides in the 4th to 1158th nucleotides of SEQ ID NO: 1, wherein at least the codon corresponding to the 955th to the 957th nucleotides of SEQ ID NO: 1 is converted to a codon encoding an amino acid other than glutamic acid;
(c) a nucleotide sequence having an identity of 80% or more with the 4th to 1158th nucleotides of SEQ ID NO: 1, wherein at least the codon corresponding to the 955th to the 957th nucleotides of SEQ ID NO: 1 is converted to a codon encoding an amino acid other than glutamic acid; and
(d) a nucleotide sequence hybridizable with the complementary strand of the 4th to 1158th nucleotides of SEQ ID NO: 1 under stringent conditions, wherein at least the codon corresponding to the 955th to the 957th nucleotides of SEQ ID NO: 1 is converted to a codon encoding an amino acid other than glutamic acid.

Embodiment 8: An expression vector containing the nucleic acid according to Embodiment 6 or 7.

Embodiment 9: A method of producing a recombinant protein having a β-galactoside-α2,3-sialyltransferase activity, wherein said protein is a β-galactoside-α2,3-sialyltransferase protein substantially not having any neuraminidase activity, and the method comprises:
1) transforming a host cell with an expression vector containing a nucleic acid according to Embodiment 6 or 7;
2) culturing the resulting transformed cell; and
3) isolating a protein having a β-galactoside-α2,3-sialyltransferase activity but substantially not having any neuraminidase activity from the cultured transformed cell or the culture supernatant thereof.

### ADVANTAGEOUS EFFECTS OF INVENTION

The present invention provides a novel β-galactoside-α2,3-sialyltransferase protein substantially not having any neuraminidase activity while maintaining the advantages of a β-galactoside-α2,3-sialyltransferase derived from a marine microorganism and provides a nucleic acid encoding the protein; hence, sialic acid can be efficiently transferred to a sugar chain with easier treatment than ever. Sialic acid is often located at the nonreducing terminus of a complex carbohydrate chain *in vivo* and is a very important sugar from the viewpoints of sugar chain functions. Accordingly, sialyltransferase is one of the most demanded enzymes among glycosyltransferases, and the provision of the novel sialyltransferase of the present invention meets such a high demand.

### BRIEF DESCRIPTION OF DRAWINGS

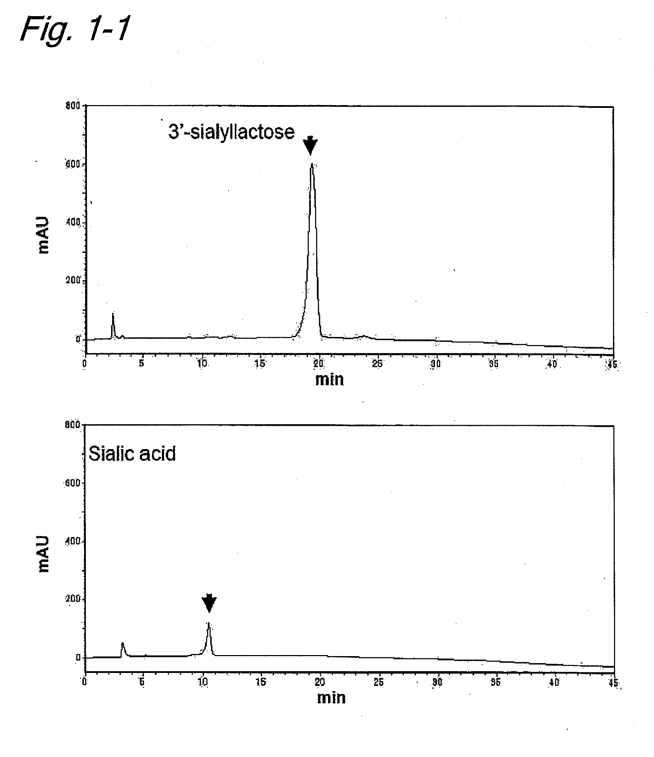

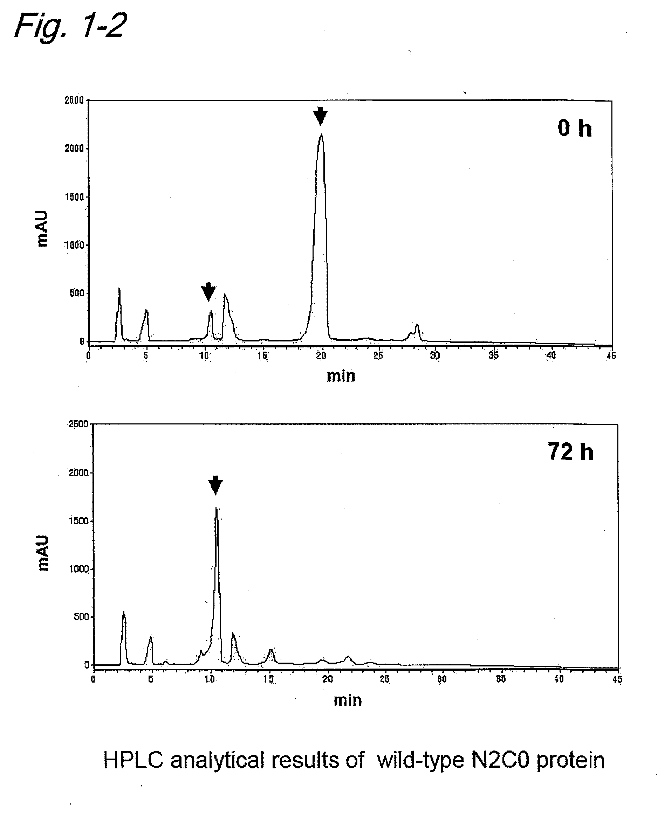

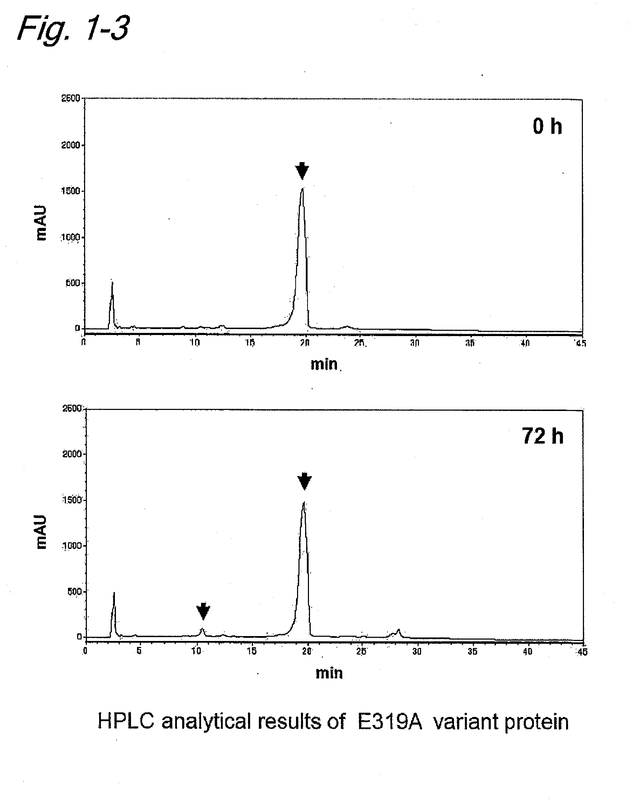

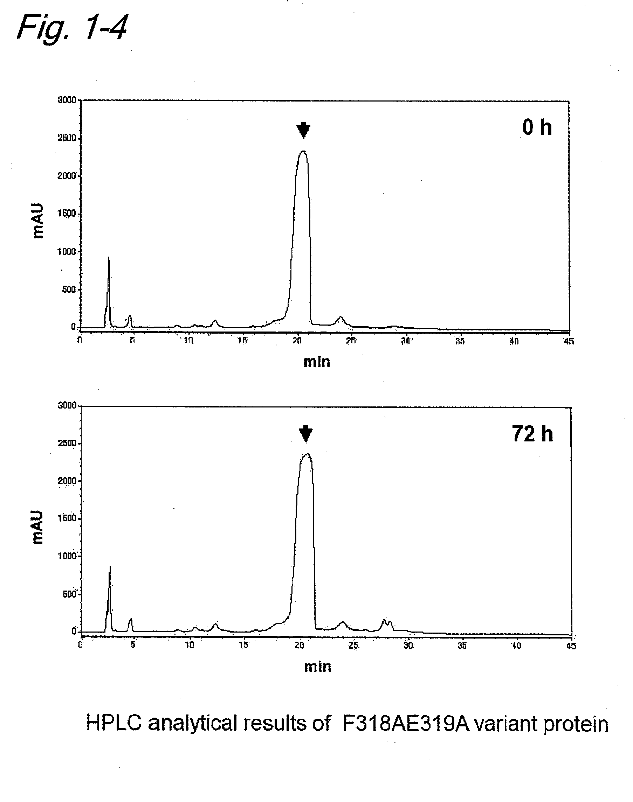

Fig. 1-1 shows HPLC analytical results of 3'-sialyllactose (3'-SL) and sialic acid (Neu5Ac).
Fig. 1-2 includes graphs showing HPLC analytical results of a reaction solution in which 3'-sialyllactose is reacted with a crude enzyme solution prepared from bacterial cells obtained by culturing *E*. *coli* cells transformed with an expression vector containing the β-galactoside-α2,3-sialyltransferase gene (SEQ ID NO: 1) derived from strain JT-ISH-467 having deletion of a leader sequence encoding a signal peptide region. The analysis was performed at 0 hour and 72 hours from the start of the reaction. The peak at a retention time of 22 minutes is assigned to 3'-sialyllactose, and the peak at a retention time of 12 minutes is to sialic acid.
Fig. 1-3 includes graphs showing HPLC analytical results of a reaction solution in which 3'-sialyllactose is reacted with a crude enzyme solution prepared from bacterial cells obtained by culturing *E*. *coli* cells transformed with an expression vector containing the β-galactoside-α2,3-sialyltransferase gene encoding a modified-type N2C0 protein (E319A variant) in which glutamic acid at position 319 in SEQ ID NO: 2 is modified to alanine. The analysis was performed at 0 hour and 72 hours from the start of the reaction. The peak at a retention time of 22 minutes is assigned to 3'-sialyllactose, and the peak at a retention time of 12 minutes is to sialic acid.
Fig. 1-4 includes graphs showing HPLC analytical results of a reaction solution in which 3'-sialyllactose is reacted with a crude enzyme solution prepared from bacterial cells obtained by culturing *E*. *coli* cells transformed with an expression vector containing the β-galactoside-α2,3-sialyltransferase gene encoding a modified-type N2C0 protein (F318AE319A variant) in which phenylalanine at position 318 and glutamic acid at position 319 in SEQ ID NO: 2 are modified to alanine. The analysis was performed at 0 hour and 72 hours from the start of the reaction. The peak at a retention time of 22 minutes is assigned to 3'-sialyllactose, and the peak at a retention time of 12 minutes is to sialic acid.

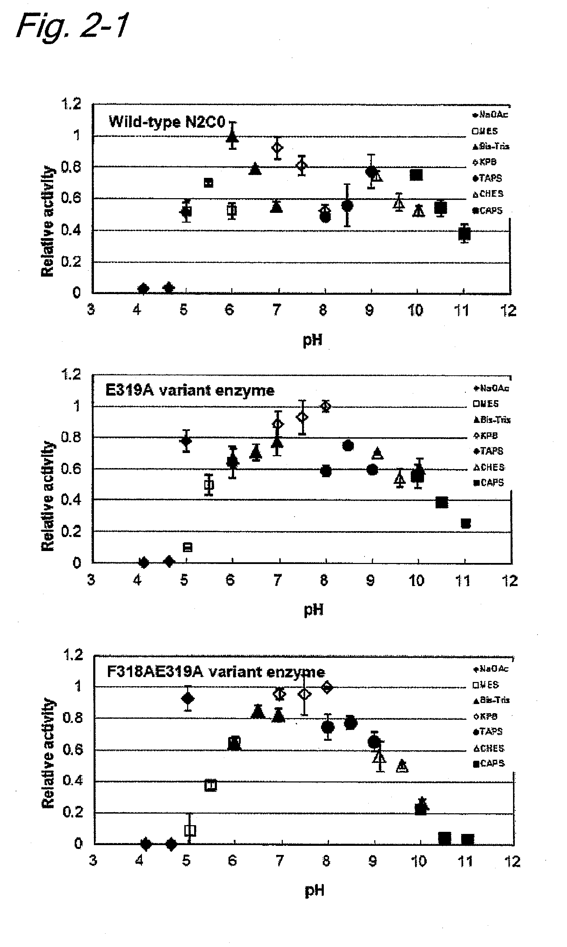

Fig. 2-1 includes graphs showing the analytical results for optimum pH of sialyltransferase activity at 30°C in each enzyme: a β-galactoside-α2,3-sialyltransferase (wild-type) derived from strain JT-ISH-467 having deletion of the signal peptide region; a modified-type β-galactoside-α2,3-sialyltransferase (E319A variant) in which glutamic acid at position 319 of SEQ ID NO: 2 is modified to alanine; and a modified-type β-galactoside-α2,3-sialyltransferase (F318AE319A variant) in which phenylalanine at position 318 and glutamic acid at position 319 in SEQ ID NO: 2 are modified to alanine.

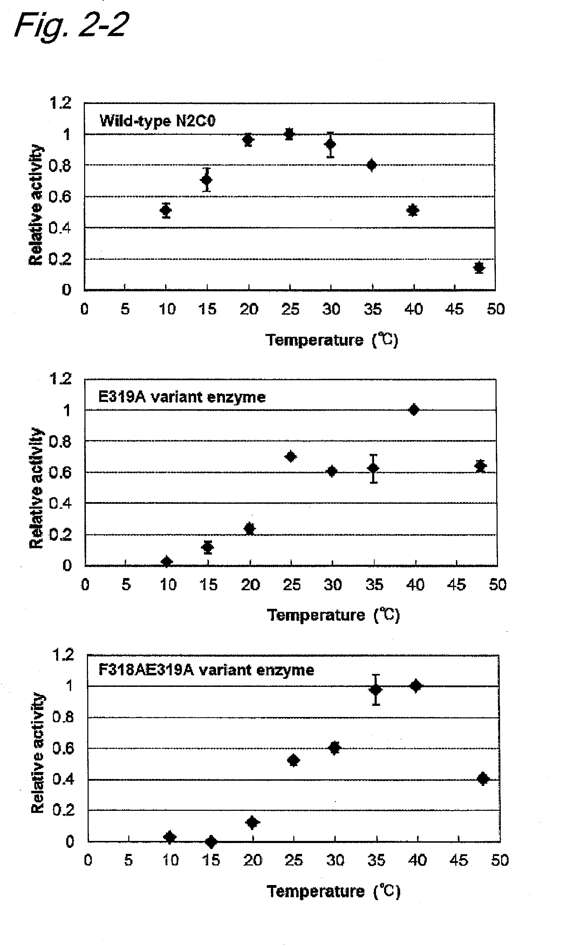

Fig. 2-2 includes graphs showing the analytical results for optimum temperature of sialyltransferase activity at pH 6.0 in each enzyme: a β-galactoside-α2,3-sialyltransferase (wild-type) derived from strain JT-ISH-467 having deletion of the signal peptide region; a modified-type β-galactoside-α2,3-sialyltransferase (E319A variant) in which glutamic acid at position 319 of SEQ ID NO: 2 is modified to alanine; and a modified-type β-galactoside-α2,3-sialyltransferase (F318AE319A variant) in which phenylalanine at position 318 and glutamic acid at position 319 in SEQ ID NO: 2 are modified to alanine.

### EMBODIMENTS OF THE INVENTION

The present invention will now be described in further detail.

### Definition

Unless otherwise specifically defined throughout the specification, the scientific terms and technical terms used in relation to the present invention are intended to have the same meanings as those generally used by those skilled in the art.

The term "isolated" for a molecule such as a protein, a nucleic acid, and an antibody throughout the specification refers to a state not substantially containing components present in the natural state of the molecule. Examples of such a state include a state of substantially not containing other molecules derived from species naturally producing the molecule, a state of expressing the molecule in cells of species different from the species naturally producing the molecule or expressing the molecule in an established culture cell system, and a state of chemically synthesizing the molecule. Furthermore, a molecule in an "isolated" state may be obtained through purification of the molecule by any known process in the art so as not to substantially contain other components present in the natural state. Throughout the specification, the term "not substantially contain" a component refers to a state in which the content of the component is reduced compared to that in the natural state. Examples of the state "not substantially contain" a component include a case not containing the component at all, a case containing the component in an amount below the detection limit, and a case having a component content reduced to 50% or less, 25% or less, 10% or less, 7% or less, 5% or less, 3% or less, 1% or less, 0.5% or less, or 0.1% or less of that in the natural state.

The term "protein" throughout the specification refers to a molecule containing at least two amino acid residues linked to each other by a peptide bond. The term "protein" throughout the specification can also be referred to as "polypeptide".

The term "nucleic acid" throughout the specification refers to a deoxyribonucleic acid (DNA) or ribonucleic acid (RNA) composed of at least two nucleotides. The term "nucleic acid" throughout the specification is also referred to as "polynucleotide". It should be understood by those skilled in the art that in the case where a nucleic acid described by the nucleotide sequence of DNA is intended to be RNA, thymine in the nucleotide sequence of the DNA is replaced by uracil.

The term "sialic acid" throughout the specification refers to a neuraminic acid derivative belonging to the sialic acid family. More specifically, it refers to, for example, *N-*acetylneuraminic acid (Neu5Ac), *N*-glycolylneuraminic acid (Neu5Gc), 5-deamino-5-hydroxyneuraminic acid (KDN), and disialic acid (di-N-acetylneuraminic acid: Neu5Acα2,8(9)Neu5Ac).

The term "β-galactoside-α2,3-sialyltransferase" throughout the specification refers to a protein having an activity to transfer sialic acid from cytidine monophosphate (CMP)-sialic acid to the 3-position of a galactose residue in a complex carbohydrate chain or a free sugar chain, to the 3-position of galactose present in an oligosaccharide such as lactose or *N-*acetyllactosamine, or to the 3-position of a monosaccharide, such as galactose, *N-*acetylgalactosamine, glucose, *N*-acetylglucosamine, or mannose, which can constitute a complex carbohydrate and has a hydroxyl group on the carbon at the 3-position. The term "β-galactoside-α2,3-sialyltransferase activity" throughout the specification refers to such activities for β-galactoside-α2,3-sialyltransferase described above. The term "β-galactoside-α2,3-sialyltransferase activity" may be also simply referred to as "sialyltransferase activity" throughout the specification.

The term "neuraminidase" throughout the specification refers to a protein having an activity to cleave sialic acid present at a nonreducing terminus of a complex carbohydrate chain or a free sugar chain, from the sugar chain. The neuraminidase is also referred to as sialidase in this technical field. The term "neuraminidase activity" throughout the specification is an activity of proteins and refers to an activity to catalyze a reaction of cleaving sialic acid present at the nonreducing terminus of a complex carbohydrate chain or a free sugar chain, from the sugar chain. The term "substantially not having any neuraminidase activity" refers to a state of a neuraminidase activity reduced than that of a wild-type protein. Examples of such a state include a case not having the neuraminidase activity at all, a case having an activity below the detection limit, and a case having a neuraminidase activity reduced to 10% or less, 7% or less, 5% or less, 3% or less, 1% or less, 0.5% or less, 0.1% or less, 0.05% or less, or 0.0 1 % or less of that of the wild-type protein.

The "vector" throughout the specification is a nucleic acid that can be used for introducing a nucleic acid linked thereto into a host cell. The term "expression vector" refers to a vector that can induce the expression of the protein encoded by the nucleic acid introduced by the vector. Examples of the vector include plasmid vectors and virus vectors.

The term "host cell" throughout the specification refers to a cell that will be transfected or transformed with a vector. The host cell can be appropriately selected by those skilled in the art depending on the vector to be used. The host cell can be derived from a prokaryote such as *Escherichia coli (E. coli)* or a cell derived from a unicellular eukaryote such as yeast or a eukaryote such as a plant cell and an animal cell (e.g., human cell, monkey cell, hamster cell, rat cell, mouse cell, or insect cell).

### N2C0 protein derived from strain JT-ISH-467

The present invention provides a novel protein having a β-galactoside-α2,3-sialyltransferase activity and substantially not having any neuraminidase activity.

The present inventors found a N0C0 protein (SEQ ID NO: 4) having a β-galactoside-α2,3-sialyltransferase activity from a marine bacterium, *Photobacterium phosphoreum* strain JT-ISH-467, and obtained a N2C0 protein (SEQ ID NO: 2) having a β-galactoside-α2,3-sialyltransferase activity by removing the signal peptide region from N0C0, and further adding methionine to the N-terminus (WO 2006/112253). This N2C0 derived from strain JT-ISH-467 is an enzyme protein having both a sialyltransferase activity and a neuraminidase activity as in the sialyltransferase derived from many other marine bacteria.

Throughout the specification, the term "N2C0" indicates a β-galactoside-α2,3-sialyltransferase derived from strain JT-ISH-467 having deletion of the signal peptide region (SEQ ID NO: 2, hereinafter also referred to as wild-type N2C0 throughout the specification) and variants thereof, unless otherwise specifically defined. In addition, amino acid sequences prepared by removing methionine at the N-terminus in SEQ ID NO: 2 and adding an appropriate signal peptide thereto are included. A variant or variant-type of N2C0 protein refers to a protein having amino acid residue variation in the amino acid sequence of SEQ ID NO: 2. Among N2C0 protein variants, in particular, a protein having an amino acid residue modified so as substantially not to have any neuraminidase activity is referred to as a mutant or a modified-type of the N2C0 protein. Accordingly, "N2C0" is used as modified-type N2C0 of the present invention and also as a collective term for the wild-type, the variant-type, and the modified-type of the present invention, depending on the context. Furthermore, since N2C0 shows a sialyltransferase activity, N2C0 may be simply referred to as "sialyltransferase" throughout the specification. The present invention provides a modified protein having a β-galactoside-α2,3-sialyltransferase activity and substantially not having any neuraminidase activity by modifying a specific amino acid residue of N2C0 protein.

N2C0 (wild-type) may be typically a protein containing the amino acid sequence of SEQ ID NO: 2 or a protein encoded by a nucleic acid containing the nucleotide sequence of SEQ ID NO: 1. Alternatively, N2C0 may be a protein being a variant of the protein containing the amino acid sequence of SEQ ID NO: 2 or the protein encoded by a nucleic acid containing the nucleotide sequence of SEQ ID NO: 1 and having a sialyltransferase activity similar to that of N2C0.

The variant of N2C0 may be a protein containing an amino acid sequence having deletion, substitution, insertion, and/or addition of one or more amino acids in the amino acid sequence of SEQ ID NO: 2. The substitution may be conservative, which indicates the replacement of a certain amino acid residue by another residue having similar physicochemical characteristics. Non-limiting examples of the conservative substitution include replacement between aliphatic group-containing amino acid residues such as Ile, Val, Leu, and Ala and replacement between polar residues such as replacements between Lys and Arg; Glu and Asp; and Gln and Asn. For example, a protein where phenylalanine (F) at position 318 of SEQ ID NO: 2 is replaced with alanine (A) is an example of the conservative substitution.

A variant derived by deletion, substitution, insertion, and/or addition of amino acid or amino acids can be produced by subjecting a DNA encoding its wild-type protein to, for example, well-known site-directed mutagenesis (see, e.g., Nucleic Acid Research, Vol. 10, No. 20, pp. 6487-6500, 1982, which is hereby incorporated by reference in its entirety). Throughout the specification, the term "one or more amino acids" indicates amino acids which can be deleted, substituted, inserted, and/or added by site-directed mutagenesis. In addition, throughout the specification, the term "one or more amino acids" optionally indicates one to several amino acids, and the number of the amino acids, which is nonlimiting, is preferably one to ten, preferably nine or less, seven or less, five or less, three or less, or two or less, and more preferably one or less.

The site-directed mutagenesis may be performed, for example, using a synthetic oligonucleotide primer that is complementary to a single-stranded phage DNA to be mutated, except for having a specific mismatch, i.e., a desired mutation. That is, a complementary strand is synthesized in the phage by using the synthetic oligonucleotide as a primer, and a host cell is transformed with the resulting double-stranded DNA. The transformed bacterial culture is plated on agar to form plaques of phage-containing single cells. As a result, in theory, 50% of new colonies contain phages having the mutation as a single strand, while the remaining 50% have the original sequence. The resulting plaques are subjected to hybridization with a synthetic probe labeled by kinase treatment at a temperature which allows hybridization with DNA completely identical to one having the above desired mutation, but not with DNA having the original strand. Subsequently, plaques hybridized with the probe are picked up and cultured to collect the DNA.

Other than the site-directed mutagenesis described above, the deletion, substitution, insertion, and/or addition of one or more amino acids in an amino acid sequence of a biologically active peptide, while retaining the activity, can also be performed through treatment of a gene with a mutagen, or through a process including selective cleavage of a gene; removal, substitution, insertion, or addition of a selected nucleotide or nucleotides; and then ligation. N2C0 in the present invention is, but not limited to, a protein composed of an amino acid sequence having deletion, substitution, or addition of one to ten, preferably nine or less, seven or less, five or less, three or less, or two or less, and more preferably one or less amino acids in SEQ ID NO: 2 and having a β-galactoside-α2,3-sialyltransferase activity.

Furthermore, the variant of N2C0 may be a protein containing an amino acid sequence having an amino acid identity of at least 80%, preferably 85% or more, 90% or more, 93% or more, 95% or more, 96% or more, 97% or more, 98% or more, or 99% or more, and more preferably 99.3% or more with the amino acid sequence of SEQ ID NO: 2. Herein, the variant of N2C0 may be a protein having a sialyltransferase activity similar to that of wild-type N2C0.

The percent identity between two amino acid sequences may be determined by visual inspection and mathematical calculation. Alternatively, the percent identity between two protein sequences may be determined by comparing sequence information based on the algorithm by Needleman, S. B. and Wunsch, C. D. (J. Mol. Biol., 48: 443-453, 1970) and using the GAP computer program available from the University of Wisconsin Genetics Computer Group (UWGCG). The preferred default parameters for the GAP program include: (1) a scoring matrix, blosum62, as described by Henikoff, S. and Henikoff, J. G. (Proc. Natl. Acad. Sci. USA, 89: 10915-10919, 1992); (2) a gap weight of 12; (3) a gap length weight of 4; and (4) no penalty for end gaps.

Other programs for sequence comparison used by those skilled in the art may also be used. The percent identity can be determined by comparing sequence information using, e.g., the BLAST program described by Altschul, et al. (Nucl. Acids. Res., 25, pp. 3389-3402, 1997). This program is available from the web sites of the National Center for Biotechnology Information (NCBI) or the DNA Data Bank of Japan (DDBJ) on the Internet. The details of various conditions (parameters) for identity search using the BLAST program are shown on these web sites, and default values are commonly used for search although the settings may be partially changed as appropriate. Alternatively, the percent identity between two amino acid sequences may be determined using a program such as genetic information processing software GENETYX Ver. 7 (Genetyx Corporation) or using an algorithm such as FASTA. In such a case, default values may be used for search.

The percent identity between two nucleic acid sequences can be determined by visual inspection and mathematical calculation. More preferably, the comparison is performed by comparing sequence information using a computer program. A typical preferred computer program is the Genetic Computer Group (GCG; Madison, WI) Wisconsin package version 10.0 program, "GAP" (Devereux, et al., 1984, Nucl. Acids Res., 12: 387). This "GAP" program can be used not only for comparison between two nucleic acid sequences but also for comparison between two amino acid sequences and between a nucleic acid sequence and an amino acid sequence. The preferred default parameters for the "GAP" program include: (1) the GCG implementation of a unary comparison matrix (containing a value of 1 for identities and 0 for non-identities) for nucleotides, and the weighted amino acid comparison matrix of Gribskov and Burgess, Nucl. Acids Res., 14: 6745, 1986, as described in Schwartz and Dayhoff, eds., "Atlas of Polypeptide Sequence and Structure", National Biomedical Research Foundation, pp. 353-358, 1979, or other comparable comparison matrices; (2) a penalty of 30 for each gap for amino acids and an additional penalty of 1 for each symbol in each gap, or a penalty of 50 for each gap for nucleotide sequences and an additional penalty of 3 for each symbol in each gap; (3) no penalty for end gaps; and (4) no maximum penalty for long gaps. Other sequence comparison programs used by those skilled in the art can also be used. For example, the BLASTN program version 2.2.7, which is available via the U.S. National Library of Medicine website: http://www.ncbl.nlm.nih.gov/blast/bl2seq/bls.html, or the UW-BLAST 2.0 algorithm can be used. Setting of the standard default parameters for the UW-BLAST 2.0 is described at the following Internet site: http://blast.wustl.edu. In addition, the BLAST algorithm uses the BLOSUM62 amino acid scoring matrix, and optional parameters that can be used are as follows: (A) inclusion of a filter to mask segments of the query sequence having low compositional complexity (determined by the SEG program of Wootton and Federhen (Computers and Chemistry, 1993); also see Wootton and Federhen, 1996, "Analysis of compositionally biased regions in sequence databases", Methods Enzymol., 266: 544-71) or segments consisting of short-periodicity internal repeats (determined by the XNU program of Claverie and States (Computers and Chemistry, 1993)), and (B) a statistical significance threshold for reporting matches against database sequences or E-score (the expected probability of matches being found merely by chance, in accordance with the statistical model (Karlin and Altschul, 1990); if the statistical significance ascribed to a match is greater than the E-score threshold, the match will not be reported.); preferred E-score threshold values are 0.5, or in order of increasing preference, 0.25, 0.1, 0.05, 0.01, 0.001, 0.0001, 1e-5, 1e-10, 1e-15, 1e-20, 1e-25, 1e-30, 1e-40, 1e-50, 1e-75, or 1e-100.

The variant of N2C0 may also be a protein encoded by a nucleic acid containing a nucleotide sequence hybridizable with the complementary strand of the nucleotide sequence of SEQ ID NO: 1 under stringent conditions. Herein, the variant of N2C0 may be a protein having a β-galactoside-α2,3-sialyltransferase activity similar to that of wild-type N2C0.

Herein, the term "under stringent conditions" refers to hybridization under moderately or highly stringent conditions. Specifically, moderately stringent conditions can be readily determined by those having ordinary skill in the art, e.g., on the basis of the length of DNA. The basic conditions are set forth by Sambrook, et al., Molecular Cloning: A Laboratory Manual, 3rd edition, chapter 6, Cold Spring Harbor Laboratory Press, 2001 and include, for example, the use of a prewashing solution of 5 × SSC containing 0.5% SDS and 1.0mM EDTA (pH 8.0), hybridization conditions at about 42°C in 2 × SSC to 6 × SSC, preferably 5 × SSC to 6 × SSC, containing about 50% formamide and 0.5% SDS (or at about 42°C in another similar hybridization solution such as Stark's solution in about 50% formamide), and washing conditions at about 50°C to 68°C in, for example, 0.1×SSC to 6 × SSC containing 0.1% SDS. Preferably, the moderately stringent conditions include hybridization conditions (and washing conditions) at about 50°C in 6 × SSC containing 0.5% SDS. Highly stringent conditions can also be readily determined by those skilled in the art, e.g., depending on the length of DNA.

In general, these conditions include hybridization at higher temperature and/or lower salt concentration than the moderately stringent conditions (for example, hybridization at about 65°C in 0.2 × SSC to 6 × SSC, preferably 6 × SSC, more preferably 2 × SSC, most preferably 0.2 × SSC or 0.1 × SSC containing about 0.5% SDS) and/or washing, and are defined to involve such hybridization conditions and washing at about 65°C to 68°C in 0.1 × SSC to 0.2 × SSC containing 0.1% SDS. The buffer solution for hybridization or washing may be SSPE (1 × SSPE contains 0.15M NaCl, 10mM NaH₂PO₄, and 1.25mM EDTA, pH 7.4) in place of SSC (1 × SSC contains 0.15M NaCl and 15mM sodium citrate). The washing is performed for about 15 minutes to 1 hour after the completion of hybridization.

A commercially available hybridization kit including a probe that does not utilize a radioactive substance can also be used. Specifically, for example, hybridization with an ECL direct labeling & detection system (manufactured by Amersham) is available. Examples of stringent hybridization involve hybridization in the hybridization buffer included in the kit to which a blocking reagent and NaCl are added in concentrations of 5% (w/v) and 0.5M, respectively, at 42°C for 4 hours and washing twice in 0.5 × SSC containing 0.4% SDS at 55°C for 20 minutes and once in 2 × SSC at room temperature for 5 minutes.

The sialyltransferase activity of a variant of N2C0 may be measured by a known method, e.g., the process described in J. Biochem., 120, 104-110 (1996) (which is hereby incorporated by reference in its entirety). For example, the enzyme activity can be evaluated by performing an enzyme reaction using *N*-acetylneuraminic acid (CMP-NeuAc) as a sugar donor substrate and lactose as a sugar acceptor substrate and evaluating the amount of the reaction product, i.e., sialyllactose. Note that one enzyme unit (1 U) of sialyltransferase is defined as the amount of the enzyme required to transfer one micromole of sialic acid per minute.

The linkage mode of sialic acid transferred to the sugar acceptor substrate can be determined by, but not limited to, any procedure known to those skilled in the art, e.g., a method using a pyridylaminated sugar chain or nuclear magnetic resonance spectroscopy (NMR) of the reaction product. The method using a pyridylaminated sugar chain involves an enzyme reaction using the pyridylaminated sugar chain as the sugar acceptor substrate. More specifically, an enzyme reaction is performed using pyridylaminated lactose (Galβ1-4Glc-PA, manufactured by Takara Bio Inc.) as a sugar acceptor substrate and CMP-NeuAc as a sugar donor substrate, and the reaction product is analyzed by high-performance liquid chromatography (HPLC). From the retention time of the reaction product, the position at which sialic acid was transferred is determined. The binding mode can be determined by any of the above-mentioned methods.

Amino acid residues that are desired not to be modified in the modified-type N2C0 protein of the present invention will be described below.

### Modified-type sialyltransferase protein

The modified-type N2C0 protein of the present invention is a protein having replacement of a specific amino acid residue in the amino acid sequence of the wild-type N2C0 protein or a variant-type N2C0 protein so as substantially not to have any neuraminidase activity.

That is, the modified-type N2C0 protein of the present invention may be a protein containing an amino acid sequence selected from the group consisting of:
(a) an amino acid sequence composed of 2nd to 386th amino acids of SEQ ID NO: 2 having replacement of the glutamic acid residue at position 319 of SEQ ID NO: 2 with an amino acid other than glutamic acid;
(b) an amino acid sequence having deletion, substitution, insertion, and/or addition of one or more amino acids in an amino acid sequence composed of 2nd to 386th amino acids of SEQ ID NO: 2, wherein at least the amino acid residue corresponding to the glutamic acid residue at position 319 of SEQ ID NO: 2 is replaced with an amino acid other than glutamic acid;
(c) an amino acid sequence having an identity of 80% or more with 2nd to 386th amino acids of SEQ ID NO: 2, wherein at least the amino acid residue corresponding to the glutamic acid residue at position 319 of SEQ ID NO: 2 is replaced with an amino acid other than glutamic acid; and
(d) an amino acid sequence encoded by a nucleotide sequence hybridizable with the complementary strand of 4th to 1158th nucleotides of SEQ ID NO: 1 under stringent conditions, wherein at least the codon corresponding to the 955th to the 957th nucleotides of SEQ ID NO: 1 is converted to a codon encoding an amino acid other than glutamic acid. The modified-type N2C0 protein of the present invention may be a β-galactoside-α2,3 - sialyltransferase protein substantially not having any neuraminidase activity.

Furthermore, a methionine residue, the 1 st to 24th amino acids of SEQ ID NO: 4, or a signal peptide such as a yeast α-factor signal leader may be added to the N-terminus of the amino acid sequence specified in any of the items (a) to (d).

The term "amino acid other than glutamic acid" refers to a natural or unnatural amino acid other than glutamic acid. The amino acid other than glutamic acid is preferably a natural L-amino acid other than glutamic acid.

In a preferred embodiment, the modified-type N2C0 protein of the present invention has substitution of the amino acid residue corresponding to the glutamic acid at position 319 of SEQ ID NO: 2 to a neutral amino acid residue or a basic amino acid residue. In a more preferred embodiment, the modified-type N2C0 protein of the present invention has substitution of the amino acid residue corresponding to the glutamic acid at position 319 of SEQ ID NO: 2 to a neutral amino acid residue.

The neutral amino acid residue is asparagine, serine, glutamine, threonine, glycine, tyrosine, tryptophan, cysteine, methionine, proline, phenylalanine, alanine, valine, leucine, or isoleucine. The basic amino acid residue is lysine, arginine, or histidine. In the modified-type N2C0 protein, the neutral amino acid residue in the substitution of the amino acid residue at position 319 of SEQ ID NO: 2 is more preferably glycine, alanine, valine, leucine, isoleucine, serine, threonine, cysteine, or methionine; more preferably glycine, alanine, valine, serine, or cysteine; and most preferably alanine, valine, or serine.

Such modified-type N2C0 has a β-galactoside-α2,3-sialyltransferase activity, but substantially does not have any neuraminidase activity.

The neuraminidase activity can be measured by a known method. For example, the enzyme activity can be evaluated by hydrolyzing sialic acid from sialic acid-containing sugar chains through the effect of neuraminidase and determining the amount of the reaction product, i.e., the amount of the sugar chains from which sialic acid was released or the amount of free sialic acid. Note that one enzyme unit (1 U) of neuraminidase is defined as the amount of the enzyme required to release one micromole of sialic acid per minute.

### Modification process of amino acid

The modification of amino acid of N2C0 for preparing a modified-type N2C0 protein of the present invention can be performed by any known mutation of an amino acid sequence without specific limitation. Preferably, mutation is performed for modification in order to prepare a nucleotide sequence of a nucleic acid encoding a modified protein of the present invention.

For example, in order to modify an amino acid at a specific position of an amino acid sequence, for example, a method of using PCR can be used (Higuchi et al., (1988) Nucleic Acid Res., 16: 7351-7367; and Ho et al., (1989) Gene, 77: 51-59). That is, a target mutant can be prepared by producing a DNA encoding the target mutant through PCR using a primer containing a mismatched codon for target mutation and expressing the DNA.

A mutant by deletion, substitution, insertion, and/or addition of amino acid or acids can be produced by subjecting a DNA encoding its wild-type protein to well-known site-directed mutagenesis described above.

### Desired amino acid residue not to be modified in modified-type N2C0

The amino acid residue modification in the modified-type N2C0 protein of the present invention is performed such that the modified-type N2C0 maintains the β-galactoside-α2,3-sialyltransferase activity.

Incidentally, the inventors have specified motif sequences (YDDGS motif, FKGHP motif, and SS motif) that are highly conserved and are believed to be important for the sialyltransferase activity in wild-type N2C0 by a comparative study with the sequence of sialyltransferase derived from other marine bacteria (Yamamoto et al., (2008), BBRC, 365: 340-343). Accordingly, in SEQ ID NO: 2, Y123, D124, D125, G126, and S127 (YDDGS motif), F291, K292, G293, H294, and P295 (FKGHP motif), and S336 and S337 (SS motif) are desirably not modified. Alternatively, in the case of modifying these amino acids, it is desirable that the amino acid is modified into an amino acid having a similar property or structure such that the sialyltransferase activity is maintained. For example, tyrosine (Y) is modified to tryptophan (W) or phenylalanine (F) and more preferably phenylalanine (F); aspartic acid (D) is modified to glutamic acid (E); glycine (G) is modified to alanine (A) or valine (V) and more preferably alanine (A); serine (S) is modified to threonine (T); phenylalanine (F) is modified to tryptophan (W) or tyrosine (Y) and more preferably tyrosine (Y); lysine (K) is modified to arginine (R); histidine (H) is modified to lysine (K) or arginine (R) and more preferably arginine (R); and proline (P) is modified to valine (V) or isoleucine (I) and more preferably valine (V).

### Nucleic acid encoding modified-type N2C0 protein

The present invention provides a nucleic acid encoding the modified-type N2C0 protein of the present invention. The nucleotide sequence of such a nucleic acid is prepared by modifying the nucleotide sequence (SEQ ID NO: 1) of N2C0 to a nucleotide sequence encoding a modified amino acid of the modified-type N2C0 protein.

That is, the nucleic acid encoding the modified-type N2C0 protein of the present invention may be a nucleic acid containing a nucleotide sequence selected from the group consisting of:
(a) a nucleotide sequence composed of the 4th to 1158th nucleotides of SEQ ID NO: 1 having a codon encoding an amino acid other than glutamic acid in place of the codon of the 955th to the 957th nucleotides of SEQ ID NO: 1;
(b) a nucleotide sequence having deletion, substitution, insertion, and/or addition of one or more nucleotides in the 4th to 1158th nucleotides of SEQ ID NO: 1, wherein at least the codon corresponding to the 955th to the 957th nucleotides of SEQ ID NO: 1 is converted to a codon encoding an amino acid other than glutamic acid;
(c) a nucleotide sequence having an identity of 80% or more with the 4th to 1158th nucleotides of SEQ ID NO: 1, wherein at least the codon corresponding to the 955th to the 957th nucleotides of SEQ ID NO: 1 is converted to a codon encoding an amino acid other than glutamic acid; and
(d) a nucleotide sequence hybridizable with the complementary strand of the 4th to 1158th nucleotides of SEQ ID NO: 1 under stringent conditions, wherein at least the codon corresponding to the 955th to the 957th nucleotides of SEQ ID NO: 1 is converted to a codon encoding an amino acid other than glutamic acid. The nucleic acid encoding the modified-type N2C0 protein of the present invention may be an isolated nucleic acid encoding a β-galactoside-α2,3-sialyltransferase protein substantially not having any neuraminidase activity.

The nucleic acid of the present invention may contain a codon encoding methionine at the N-terminus, a leader sequence corresponding to nucleotides 1 to 72 of SEQ ID NO: 3, or a leader sequence derived from another organism, such as yeast α-factor leader.

In the items (a) to (d) mentioned above, the "amino acid other than glutamic acid" is, as described in the paragraph "modified sialyltransferase protein", a natural L-amino acid other than glutamic acid, preferably a neutral amino acid or basic amino acid, and more preferably a neutral amino acid.

### Method of producing the protein of the present invention

The present invention also relates to a method of producing a modified-type N2C0 protein of the present invention. In a preferred embodiment, the method of the present invention produces the protein of the present invention.

### (1) Method of producing recombinant protein

The present invention provides an expression vector containing a nucleic acid of the present invention and a host cell containing the expression vector. Moreover, the present invention also provides a method of producing a recombinant modified-type N2C0 protein by culturing the host cell containing the expression vector under conditions suitable for expressing the recombinant protein and collecting the expressed recombinant protein.

In order to produce the recombinant protein of the present invention, a nucleic acid sequence encoding a modified-type N2C0 protein and being operably linked to a suitable transcription or translation regulatory nucleotide sequence derived from a gene of mammalian, microorganism, viral, insect, or other origin is inserted into an expression vector chosen depending on the host to be used. Examples of the regulatory sequence include a transcription promoter, an operator, an enhancer, an mRNA ribosome binding site, and suitable sequences regulating the initiation and termination of transcription and translation.

The nucleic acid sequence encoding the modified-type N2C0 protein to be inserted into the vector of the present invention is a nucleotide sequence of the above-described nucleic acid of the present invention. The sequence may include a leader sequence or not. If the nucleotide sequence includes a leader sequence, the leader sequence may correspond to nucleotides 1 to 72 of SEQ ID NO: 3 or may be replaced by a leader sequence derived from another organism. An expression system can be designed such that the expressed protein is secreted to the outside of the host cells by replacing the leader sequence.

The recombinant modified-type N2C0 protein of the present invention can also be expressed as a fusion protein by inserting, into a vector, a nucleic acid construct comprising a nucleic acid encoding the protein linked to a nucleic acid encoding, for example, a His tag, a FLAG^{™} tag (tag containing an amino acid sequence: DYKDDDDK (SEQ ID NO: 5)), or glutathione-S-transferase. The enzyme of the present invention can be readily purified and detected by expressing the enzyme as such a fusion protein.

Examples of the host cell suitable for expressing the protein of the present invention include prokaryotic cells, yeast, and higher eukaryotic cells. Examples of cloning and expression vectors suitable for use in bacterial, fungal, yeast, and mammalian host cells are described, for example, in Pouwels, et al., Cloning Vectors: A Laboratory Manual, Elsevier, New York, (1985) (which is hereby incorporated by reference in its entirety).

The prokaryotes include Gram-negative and Gram-positive bacteria such as *E. coli* or *Bacillus subtilis.* For a prokaryotic cell such as *E. coli* used as a host, the protein of the present invention may be designed to have an N-terminal methionine residue for the purpose of facilitating the expression of a recombinant polypeptide within prokaryotic cells. This N-terminal methionine can be removed from the expressed recombinant protein.

Expression vectors to be used in prokaryotic host cells generally contain one or more phenotype selectable marker genes. Such a phenotype selectable marker gene is, for example, a gene imparting antibiotic resistance or auxotrophy. Examples of expression vectors suitable for prokaryotic host cells include commercially available plasmids such as pBR322 (ATCC37017) or derivatives thereof. The pBR322 contains genes for ampicillin and tetracycline resistance, and thereby transformed cells can be easily identified. DNA sequences of a suitable promoter and a nucleic acid encoding a modified-type N2C0 protein are inserted into this pBR322 vector. Other examples of commercially available vectors include pKK223-3 (Pharmacia Fine Chemicals, Inc. Uppsala, Sweden) and pGEM1 (Promega Biotech AB, Madison, Wisconsin, United States).

Examples of promoter sequences usually used in expression vectors for prokaryotic host cells include tac promoters, β-lactamase (penicillinase) promoters, and lactose promoters (Chang et al., Nature, 275: 615, 1978; and Goeddel et al., Nature, 281: 544, 1979, which are hereby incorporated by reference in their entirety).

Alternatively, the recombinant protein of the present invention may be expressed in yeast host cells. Saccharomyces (e.g., *S*. *cerevisiae*) is preferably used, but other genera of yeast, such as Pichia or Kluyveromyces, may also be used. Yeast vectors often contain a sequence of replication origin from a 2µ yeast plasmid, an autonomously replicating sequence (ARS), a promoter region, a sequence for polyadenylation, a sequence for transcription termination, and a selectable marker gene. A yeast α-factor leader sequence can also be used to induce secretion of a modified-type N2C0 protein. There are also known other leader sequences that are suitable for facilitating recombinant polypeptide secretion from yeast hosts. A method of transforming yeast is described, for example, in Hinnen et al., Proc. Natl. Acad. Sci. USA, 75: 1929-1933, 1978 (which is hereby incorporated by reference in its entirety).

The recombinant protein of the present invention can also be expressed using a mammalian or insect host cell culture system. Established cell lines of mammalian origin can also be used. Transcription and translation control sequences for mammalian host cell expression vectors can be obtained from viral genomes. Promoter and enhancer sequences usually used are induced from, for example, polyoma virus or adenovirus 2. Other gene elements for expressing structural gene sequences in mammalian host cells may also be provided by using DNA sequences induced from the SV40 viral genome, e.g., SV40 origin, early and late promoters, enhancers, splice sites, and polyadenylation sites. Vectors for use in mammalian host cells can be constructed by, for example, the method of Okayama and Berg (Mol. Cell. Biol., 3: 280, 1983, which is hereby incorporated by reference in its entirety).

One method of producing a recombinant modified-type N2C0 protein of the present invention includes culturing host cells transformed with an expression vector containing a nucleic acid sequence encoding the protein under conditions allowing expression of the protein. Then, the protein is collected from the culture medium or cell extract in a manner suitable for the expression system used.

The procedure for purifying the recombinant modified-type N2C0 protein is appropriately selected depending on such factors as the type of the host to be used and the secretion of the protein of the present invention into the culture medium. Examples of the procedure for purifying the recombinant protein include column chromatographic approaches using, for example, an anion exchange column, a cation exchange column, a gel filtration column, a hydroxyapatite column, a CDP-hexanolamine agarose column, a CMP-hexanolamine agarose column, or a hydrophobic column; Native-PAGE; and combinations thereof. Alternatively, for the expression of the recombinant protein in a form fused with a tag or the like for easy purification, affinity chromatography may be used for purification. For example, fusion protein with a histidine tag, a FLAG^{™} tag, or glutathione-S-transferase (GST) can be purified by affinity chromatography using a nitrilotriacetic acid (Ni-NTA) column, an anti-FLAG antibody-bound column, or a glutathione-bound column, respectively.

Although the recombinant modified-type N2C0 protein may be purified to give an electrophoretically single band, the modified-type N2C0 protein of the present invention may be of a completely purified or partially purified form because it has sufficient activity even in a partially purified form.

The present invention will now be described in more detail by way of examples, which should not be construed as limiting the technical scope of the invention. Based on the description in the specification, modifications and limitations will be apparent to those skilled in the art, and such modifications and limitations fall within the technical scope of the invention.

### EXAMPLES

### Example 1: Preparation of variant-type β-galactoside-α2,3-sialyltransferase

### (1) Preparation of ISH467-N2C0 variant

Variants of β-galactoside-α2,3-sialyltransferase N2C0 (Patent Literature 3: WO 2006/112253) derived from *Photobacterium phosphoreum* strain JT-ISH-467 were produced by replacement of amino acid or amino acids. PCR (1st PCR) was performed using ISH467-N2C0/pTrc99A (Patent Literature 3: WO 2006/112253), which was a template produced by the inventors. Tables 1 and 2 show primer sequences and the combinations thereof used in the reaction. In 50 µL of a reaction solution containing a plasmid, 5 µL of 10 x PyroBest buffer, 4 µL of 2.5mM dNTP, 5pM of each primer, and 0.5 µL of PyroBest DNA polymerase (manufactured by Takara Bio Inc.), PCR was carried out at 96°C for 3 min once, 96°C for 1 min, 55°C for 1 min, and 72°C for 2 min 15 cycles, and 72°C for 1 min once (1st PCR). Table 2 shows the primer sets used in the PCR. The resulting PCR products were subjected to agarose electrophoresis in TAE buffer using a low melting point agarose (SeaPlaqueGTG). Each gel piece containing a DNA fragment was cut out, and an equiamount of 200mM NaCl was added to the gel piece, followed by treatment at 70°C for 10 min to melt the gel. This sample was extracted once with phenol, once with phenol/chloroform, and then once with chloroform, followed by ethanol precipitation to collect DNA fragments. Reaction solutions were prepared as in the 1st PCR using the resulting 5'-side fragment and 3'-side fragment as templates, and fusion-PCR (2nd PCR) was carried out using the Fw primer used in the amplification of the 5'-side fragment and the Rv primer used in the amplification of the 3'-side fragment. The 2nd PCR products were also subjected to electrophoresis and then purification as described above. The variants produced included PCR products each to which His × 6-Tag was added using ISH467-23ST-C0-His-BamHI shown in Table 1.

[Table 1-1]

**Table 1 Primers used in production of variant-type β-galactoside-α2,3-sialyltransferase**

| Name | Sequence (5'-3') | Length |
|---|---|---|
| 467-23ST-N2-Nco | GGGCTGTA CCATGG ACTCTAAGCACAATAACTCAG (SEQ ID NO: 6) | 35-mer |
| ISH467-23ST-C0-BamHI | CTTAGAAT GGATCC TTACTGCAAATCACTTATCAAC (SEQ ID NO: 7) | 36-mer |
| ISH467-23ST-C0-His-BamHI | GGGG GGATCC TTAGTGATGGTGATGGTGATG CTGCAAATCACTTATCAACTTAACATCATT (SEQ ID NO: 8) | 61-mer |
| ISH467-23ST-F318A-F | AAATTTATAATAAGATACCAGCTGAAGCTCTAAT AATGACTGATGC (SEQ ID NO: 9) | 46-mer |
| ISH467-23ST-F318A-R | GCATCAGTCATTATTAGAGCTTCAGCTGGTATCT TATTATAAATTT (SEQ ID NO: 10) | 46-mer |
| ISH467-23ST-E319A-F | AAATTTATAATAAGATACCATTTGCAGCTCTAAT AATGACTGATGC (SEQ ID NO: 11) | 46-mer |
| ISH467-23ST-E319A-R | GCATCAGTCATTATTAGAGCTGCAAATGGTATCT TATTATAAATTT (SEQ ID NO: 12) | 46-mer |
| ISH467-23ST-F318AE319A-F | AAATTTATAATAAGATACCAGCTGCAGCTCTAAT AATGACTGATGC (SEQ ID NO: 13) | 46-mer |
| ISH467-23ST-F318AE319A-R | GCATCAGTCATTATTAGAGCTGCAGCTGGTATCT TATTATAAATTT (SEQ ID NO: 14) | 46mer |
| ISH467-23ST-S337A-F | CTGTCGGTGGAATGGGAAGTGCTGTATTTTTTAG CTTGCCAAA (SEQ ID NO: 15) | 43-mer |
| ISH467-23ST-S337A-R | TTTGGCAAGCTAAAAAATACAGCACTTCCCATTC CACCGACAG (SEQ ID NO: 16) | 43-mer |

**[Table 1-2]**

| Name | Sequence (5'-3') | Length |
|---|---|---|
| ISH467-23ST-E319V-F | AAATTTATAATAAGATACCATTTGTGGCTCTAAT AATGACTGATGC (SEQ ID NO: 17) | 46-mer |
| ISH467-23ST-E319V-R | GCATCAGTCATTATTAGAGCCACAAATGGTATCT TATTATAAATTT (SEQ ID NO: 18) | 46-mer |
| ISH467-33ST-E319S-F | AAATTTATAATAAGATACCATTTAGCGCTCTAAT AATGACTGATGC (SEQ ID NO: 19) | 46-mer |
| ISH467-23ST-E319S-R | GCATCAGTCATTATTAGAGCGCTAAATGGTATCT TATTATAAATTT (SEQ ID NO: 20) | 46-mer |
| ISH467-23ST-E319K-F | AAATTTATAATAAGATACCATTTAAAGCTCTAAT AATGACTGATGC (SEQ ID NO: 21) | 46-mer |
| ISH467-23ST-E319K-R | GCATCAGTCATTATTAGAGCTTTAAATGGTATCT TATTATAAATTT (SEQ ID NO: 22) | 46-mer |
| ISH467-23ST-E319H-F | AAATTTATAATAAGATACCATTTCATGCTCTAAT AATGACTGATGC (SEQ ID NO: 23) | 46-mer |
| ISH467-23ST-E319H-R | GCATCAGTCATTATTAGAGCATGAAATGGTATCT TATTATAAATTT (SEQ ID NO: 24) | 46-mer |

| | | |
|---|---|---|
| * The underline indicates a restriction enzyme site, the wavy line indicates His × 6-tag, and the boldface indicates initiation and termination codons. Substituted codons are indicated by shading. | | |

**[Table 2]**

| Table 2 Primer sets used in 1st PCR | | | |
|---|---|---|---|
| | Produced variant | 5'-side fragment (Rv primer) | 3'-side primer (Fw primer) |
| 1 | ISH467-N2CO-F318A | ISH467-23ST-F318A-R | ISH467-23ST-F318A-F |
| 2 | ISH467-N2C0-E319A | ISH467-23ST-E319A-R | ISH467-23ST-E319A-F |
| 3 | ISH467-N2C0-F318A E319A | ISH467-23ST-F318AE319A-R | ISH467-23ST-F318AE319A-F |
| 4 | ISH467-N2C0-S337A | ISH467-23ST-S337A-R | ISH467-23ST-S337A-F |
| 5 | ISH467-N2C0-F318A E319A S337A | ISH467-23ST-S337A-R | ISH467-23ST-S337A-F |
| 6 | ISH467-N2C0-E319V | ISH467-23ST-E319V-R | ISH467-23ST-E319V-F |
| 7 | ISH467-N2C0-E319S | ISH46-23ST-E319S-R | ISH467-23ST-E319S-F |
| 8 | ISH467-N2CO-E319K | ISH467-23ST-E319K-R | ISH467-23ST-E319K-F |
| 9 | ISH467-N2C0-E319H | ISH467-23ST-E319H-R | ISH467-23ST-E319H-F |

| | | | |
|---|---|---|---|
| In 1st PCR. 467-23ST-N2-Nco and ISH467-23ST-C0-BamHI (partially, ISH467-23ST-C0-His-BamHI) were respectively used as the Fw primer of the 5'-side fragment and the Rv primer of the 3'-side fragment. In 2nd PCR, 467-23ST-N2-Nco and ISH467-23ST-C0-BamHI (partially, ISH467-23ST-C0-His-BamHI) were respectively used as the Fw primer and the Rv primer. In PCR using set 5. ISH467-N2C0-F318A E319A/TOPO in set 3 was used as the template. | | | |

A specific example of PCR will now be described. In the 1st PCR of variant ISH467-23ST-E319A, the 5'-side fragment was amplified using ISH467-23ST-N2-Nco and ISH467-23ST-E319A-R as the Fw primer and the Rv primer, respectively; and the 3'-side fragment was amplified using ISH467-23ST-E319A-F and ISH467-23ST-CO-Bam (partially, ISH467-23ST-C0-His-BamHI) as the Fw primer and the Rv primer, respectively. In the 2nd PCR, ISH467-23ST-N2-Nco and ISH467-23ST-C0-Bam (partially, ISH467-23ST-C0-His-BamHI) were used as the Fw primer and the Rv primer, respectively.

These 2nd PCR fragments were each cloned into vector pCR4BluntTOPO (manufactured by Invitrogen Corp.). Ligation was carried out in accordance with the instructions attached to the vector kit. A plasmid was introduced into *E*. *coli* TB1 by electroporation, and the plasmid DNA was extracted in a usual manner (Sambrook, et al., 1989, Molecular Cloning, A laboratory manual, 2nd edition). Clones confirmed to have the insert were each analyzed to determine the nucleotide sequence of the PCR product from both ends using M13 primers (manufactured by Takara Bio Inc.) with an ABI PRISM fluorescent sequencer (Model 310 Genetic Analyzer, manufactured by Perkin Elmer, Inc.).

Variant-type N2C0/pCR4BluntTOPO was digested with NcoI and BamHI, followed by purification by electrophoresis using a low melting point agarose. A vector was similarly prepared by digesting pTrc99A with restriction enzymes NcoI and BamHI and cutting out a band of approximately 3 kb from the electrophoresis gel. The DNA fragment and the vector after restriction enzyme treatment were ligated to each other using a Ligation kit (Takara Bio Inc.). The ligation product was transformed into *E*. *coli* TB1, and a clone containing the insert gene was determined by colony PCR. The plasmid DNA was extracted in a usual manner, and the entire recombinant nucleotide sequence was determined with an ABI PRISM fluorescent sequencer. In this regard, pTrc99A FW5 and pTrc99A RV3 primers were used. Sequences of pTrc99A FW5 and pTrc99A RV3 primers are as follows:
pTrc99A FW5: 5'-GGAAGCTGTGGTATGGCTG-3' (19-mer: SEQ ID NO: 25), and
pTrc99A RV3: 5'-CGTTTCACTTCTGAGTTCGG-3' (20-mer: SEQ ID NO: 26).

Thus, the following variants of ISH467-N2C0 were prepared: ISH467-N2C0-F318A (hereinafter, simply referred to as F318A), ISH467-N2C0-E319A (hereinafter, simply referred to as E319A), ISH467-N2C0-F318AE319A (hereinafter, simply referred to as F318AE319A), ISH467-N2C0-S337A (hereinafter, simply referred to as S337A), ISH467-N2C0-F318AE319AS337A (hereinafter, simply referred to as F318AE319AS337A), ISH467-N2C0-E319V (hereinafter, simply referred to as E319V), ISH467-N2C0-E319S (hereinafter, simply referred to as E319S), ISH467-N2C0-E319K (hereinafter, simply referred to as E319K), and ISH467-N2C0-E319H (hereinafter, simply referred to as E319H), where, for example, E319A indicates that glutamic acid at position 319 of wild-type N2C0 (SEQ ID NO: 2) is replaced with alanine. Other variants are designated in the same manner.

### (2) Expression and purification of variant-type N2C0 protein containing His × 6-Tag

Four *E*. *coli* strains containing variant-type N2C0 (F318A, E319A, F318AE319A, or S337A) having His × 6-Tag prepared in the step (1) were inoculated in 50 mL of an LB medium containing ampicillin at a final concentration 100 µg/mL and were shake-cultured at 30°C overnight. The collected cells were gently suspended in 1.6 mL of 20mM Bis-Tris buffer (pH 6.0) containing 10 to 20mM imidazole, 300mM NaCl, and 0.3% Triton X-100 (hereinafter, referred to as "Lysis buffer") and were sonicated under ice cooling, followed by centrifugation. The supernatant was collected, and 3.4 mL of Lysis buffer was further added thereto to give a total amount of 5 mL. The resulting solution was divided into two fractions, and each of them was incubated in 250 µL of a Ni-NTA agarose (manufactured by QIAGEN) equilibrated with Lysis buffer in advance at 4°C for 1 hour with rotation. The agarose after the incubation was allowed to pass through an open column and was washed with 2.5 mL of 20mM Bis-Tris buffer (pH 6.0) containing 20 to 40mM imidazole, 300mM NaCl, and 0.3% Triton X-100 (hereinafter, referred to as "Wash buffer") four times, followed by elution with 0.5 mL of 20mM Bis-Tris buffer (pH 6.0) containing 250mM imidazole, 300mM NaCl, and 0.3% Triton X-100 (hereinafter, referred to as "Elution buffer") three times to purify a variant-type N2C0 protein containing His × 6-Tag.

### (3) Expression of ISH467-N2C0 variant not containing His × 6-Tag

A single colonyl of each of six *E*. *coli* strains containing variant-type N2C0 (E319A, F318AE319A, E319H, E319K, E319S, and E319V modified-type N2C0) prepared in the step (1) was inoculated in an LB medium (5 mL) containing an antibiotic, ampicillin (final concentration: 100 µg/mL) and was pre-cultured at 30°C until the absorbance at 600 nm reached about 0.5. IPTG (Isopropyl-β-D(-)-thiogalactopyranoside, manufactured by Wako Pure Chemical Industries, Ltd.) was added to the medium at a final concentration of 1mM, followed by shake culture at 30°C for 4 hours. The cells in 2 mL of the culture solution were collected by centrifugation. These cells were suspended in 200 µL of 20mM Bis-Tris buffer (pH 7.0) containing 0.3% Triton X-100 and 0.5M sodium chloride and sonicated under ice cooling. The resulting homogenate was subjected to measurement of activity as a crude enzyme solution.

### (4) Expression and purification of variant-type N2C0 variant not containing His × 6-Tag

Cells were collected with a loop from colonies of *E*. *coli* TB1 having expression vector pTrc99A containing the variant-type N2C0 clone which had been subcultured on an LBAmp plate medium and were inoculated into 10 mL of 6 mL-LB liquid medium supplemented with 30 µL of × 200 ampicillin (400 mg/20 mL) and shake-cultured at 30°C at 180 rpm for 8 hours.

Main culture was performed by the following procedure: 300 mL of LB medium supplemented with 1.5 mL of × 200 ampicillin (400 mg/20 mL) and 300 µL of 1M IPTG (1.192 g/5 mL) was placed in a 1000-mL baffle flask and was inoculated with 12 mL of the culture solution obtained above, followed by shake culturing at 30°C at 180 rpm for 24 hours. The culture solution was centrifuged to collect the cells.

The cells were suspended in 20mM Bis-Tris buffer (pH 6.0) containing 0.3% Triton X-100 and were sonicated under ice cooling. The cell homogenate was centrifuged at 4°C at 100000 × g for 1 hour to obtain the supernatant.

This crude enzyme solution was allowed to adsorb to a HiLoad 26/10 Q Sepharose HP (manufactured by Amersham) anion exchange column equilibrated with 20mM Bis-Tris buffer (pH 6.0) containing 0.3% Triton X-100 and was eluted by a linear gradient from 20mM Bis-Tris buffer (pH 6.0) containing 0.3% Triton X-100 to the buffer containing 1M sodium chloride to collect an enzymatically active fraction eluted at around 0.25M sodium chloride concentration.

The collected fraction was diluted with 20mM phosphate buffer (pH 6.0) and was allowed to adsorb to hydroxyapatite (manufactured by Bio-Rad Laboratories, Inc.) equilibrated in advance with 20mM phosphate buffer (pH 6.0) containing 0.3% Triton X-100, followed by elution with a linear gradient from 20mM phosphate buffer (pH 6.0) containing 0.3% Triton X-100 to 500mM phosphate buffer (pH 6.0) containing 0.3% Triton X-100 to thereby collect an enzymatically active fraction eluted at around 130mM phosphate buffer concentration.

The fraction was allowed to adsorb to an MonoQ 5/50 GL (manufactured by Amersham) anion exchange column and was eluted by a linear gradient from 20mM Bis-Tris buffer (pH 6.0) containing 0.3% Triton X-100 to the buffer containing 1M sodium chloride to collect an enzymatically active fraction eluted at around 0.25M sodium chloride concentration to yield a purified variant-type N2C0 protein not containing His × 6-Tag.

### Example 2: Measurement of variant-type β-galactoside-α2,3-sialyltransferase activity

### (1) Measurement of sialyltransferase activity of variant-type N2C0 containing His × 6-Tag

The β-galactoside-α2,3-sialyltransferase activities of variant-type N2C0 proteins (four variants: F318A, E319A, F318AE319A, and S337A) each containing His × 6-Tag purified in Example 1 (2) were measured. The sialyltransferase activity was measured by the method as described in J. Biochem., 120, 104-110, (1996) (hereby incorporated by reference in its entirety). Specifically, an enzyme reaction was performed using a reaction solution (30 µL) containing the enzyme prepared as described above and CMP-NeuAc (70nmol, containing about 25000 cpm CMP-NeuAc in which NeuAc was labeled with ¹⁴C; 356 cpm/mol. NeuAc represents N-acetylneuraminic acid) as a sugar donor substrate, lactose (1.25µM) as a sugar acceptor substrate, and 0.5M NaCl. The enzyme reaction was performed at 30°C for 5 minutes. After the completion of the reaction, 1.97 mL of 5mM phosphate buffer (pH 6.8) was added to the reaction solution, which was then applied to a Dowex 1 × 8 (PO₄³⁻ form, 0.2 × 2 cm, manufactured by Bio-Rad Laboratories, Inc.) column. The reaction product contained in the eluate (0 to 2 mL) from this column, i.e., the radioactivity contained in sialyllactose was measured to calculate the enzyme activity. One enzyme unit (1 U) is defined as the amount of the enzyme required to release one micromole of sialic acid per minute. The results showed the sialyltransferase activity in F318A, E319A, and F318AE319A, but not in S337A.

### (2) Measurement of neuraminidase activity of variant-type N2C0 containing His × 6-Tag (Aminoff method)

The neuraminidase activities of ISH467-N2C0 variants (four variants: F318A, E319A, F318AE319A, and S337A) each containing His × 6-Tag were measured. Specifically, 3'-sialyllactose (3'-SL) was used as a substrate for the reaction, and the sialic acid of the cleavage product was colorimetrically measured by the periodic acid-thiobarbituric acid assay (Aminoff method).

An enzyme solution was added to a reaction solution composed of 100mM sodium cacodylate buffer (pH 5.5), 500mM sodium chloride, and 13mM 3'-sialyllactose, followed by a reaction at 30°C. After the completion of the reaction, 45 µL of 1M KPB (pH 8.0) was added thereto to give a total amount of 500 µL and adjusted to pH7.0. Furthermore, 250 µL of 25mM periodic acid/0.125N H₂SO₄ was added thereto, followed by incubation at 37°C for 30 minutes. Subsequently, 200 µL of 2% sodium arsenite/0.5N HCl and 2 mL of 0.1M 2-thiobarbituric acid (pH 9.0) were mixed with the reaction solution, and the mixture was heated at 100°C for 7.5 minutes. After cooling to about room temperature, 5 mL of 5% HCl/n-butanol was mixed therewith. After light centrifugation and then being left to stand for about 15 minutes, the O.D. at 549 nm was measured.

The results showed the neuraminidase activity in variant-type N2C0 (F318A) containing His × 6-Tag, but not in three variant-types of N2C0 (E319A, F318AE319A, and S337A) containing His × 6-Tag.

As shown in the results of Examples 2 (1) and (2), a protein having a β-galactoside-α2,3-sialyltransferase activity but substantially not having any neuraminidase activity can be successfully yielded by modifying the glutamic acid at position 319 of the N2C0 protein derived from strain JT-ISH-467. The protein prepared by modifying the glutamic acid at position 319 of the N2C0 protein derived from strain JT-ISH-467 substantially does not have the neuraminidase activity, but has the β-galactoside-a2,3-sialyltransferase activity. It is therefore called a modified type protein hereinafter.

### Example 3: Property of modified-type β-galactoside-α2,3-sialyltransferase

The rate constants of modified-type N2C0 proteins (two variants: E319A and F318AE319A) not containing His × 6-Tag purified in Example 1 (4) and wild-type N2C0 protein not containing His × 6-Tag for lactose and CMP-sialic acid were calculated (Table 3). The Km and Vₘₐₓ of ISH467-N2C0 not containing His × 6-Tag for lactose and CMP-sialic acid were respectively 8.4mM and 0.72mM. The Km and Vₘₐₓ of variant E319A of ISH467-N2C0 not containing His × 6-Tag for lactose and CMP-sialic acid were 22mM and 0.53mM, respectively; and those of variant F318AE319A were 85mM and 1.59mM, respectively.

**[Table 3]**

| Table 3 Rate constants of modified-type and wild-type N2C0 proteins for lactose and CMP-sialic acid | | | |
|---|---|---|---|
| Lactose | | | |
| | *K*ₘ (mM) | *V*ₘₐₓ (nmol/min) | *k*_{cat} (/sec) |
| N2C0 enzyme | 8.4 | 3.9 | 11.2 |
| E319A variant enzyme | 22 | 12 | 99.7 |
| F318A E319A variant enzyme | 85 | 7.2 | 10.8 |

| CMP-sialic acid | | | |
|---|---|---|---|
| | *K*ₘ (mM) | *V*ₘₐₓ (nmol/min) | *k*_{cat} (/sec) |
| N2C0 enzyme | 0.72 | 7.3 | 21.0 |
| E319A variant enzyme | 0.53 | 3.2 | 106.3 |
| F318A E319A variant enzyme | 1,59 | 8.9 | 13.3 |

### Example 4: Measurement of neuraminidase activity of variant-type_β-galactoside-α2,3-sialyltransferase

The neuraminidase activities of modified-type N2C0 protein (two variants: E319A and F318AE319A) not containing His × 6-Tag and wild-type N2C0 protein not containing His × 6-Tag were measured as follows.

### (1) Neuraminidase reaction

A reaction solution for the wild-type N2C0 protein (not containing His × 6-Tag) as a control was prepared by adding an enzyme solution to a reaction solution composed of 100mM Bis-Tris buffer (pH 6.0), 500mM sodium chloride, and 40mM 3'-sialyllactose such that the final concentration of the wild-type N2C0 protein was 2 U/mL (in terms of sialyltransferase activity). A reaction solution for the modified-type N2C0 protein was prepared by adding an enzyme solution to a reaction solution composed of 100mM phosphate buffer (pH 8.0), 500mM sodium chloride, and 40mM 3'-sialyllactose such that the final concentration of the modified-type N2C0 protein was 2 U/mL (in terms of sialyltransferase activity). The reaction was performed at an optimal temperature for each sialyltransferase. The time immediately after the addition of the enzyme solution was defined as time 0, and 10 µL of the solution was sequentially sampled. Immediately after sampling, the samples were treated at 95°C for 3 minutes to terminate the reaction by deactivating the enzyme.

### (2) Quantitative analysis of neuraminidase activity by high-performance liquid chromatography (HPLC)

Acetonitrile/15mM KH₂PO₄ (pH 5.2) (75:25, V/V) of 300 µL was added to each reaction solution sequentially sampled, and 150 µL of the resulting mixture was subjected to HPLC analysis using a TSKgel Amide-80 5 µm (4.6 mm × 25 cm) column. The analysis was performed using a mobile phase A of acetonitrile/15mM KH₂PO₄ (pH 5.2) (50:50, V/V) and a mobile phase B of acetonitrile/15mM KH₂PO₄ (pH 5.2) (75:25, VN) under conditions: from 0 to 15 minutes: 100% of mobile phase B, from 15 to 45 minutes: a linear gradient of mobile phase B 100% → 0%, and from 45 to 60 minutes: 0% of mobile phase B. The absorbance was measured at 195 nm.

The results are shown in Figs. 1-1 to 1-4. As shown in the drawings, 3'-SL (3'-sialyllactose) added to the reaction system disappeared at 72 hours from the start of the reaction with the wild-type N2C0 protein, whereas almost the whole quantities of the 3'-SL added to the reaction systems remained even after 72 hours from the start of the reaction with two modified-type N2C0 proteins E319A and F318AE319A. These results demonstrate that the neuraminidase activities of two modified-type ISH467-N2C0 E319A and F318AE319A were vanished or drastically decreased.

### Example 5: Optimal pH and temperature of modified-type β-galactoside-α2,3-sialyltransferase

The optimal pH and temperature of modified-type β-galactoside-α2,3-sialyltransferases (E319A and F318AE319A) were investigated using the enzymes purified in Example 1 (4).

### (1) Optimal pH for enzyme activity of modified-typeβ-galactoside-α2,3-sialyltransferase (E319A and F318AE319A)

Acetate buffer (pH 4-5), MES buffer (pH 5-6), Bis-Tris buffer (pH 6-7), phosphate buffer (pH 6-9.5), TAPS buffer (pH 8-9), CHES buffer (pH 9-10), and CAPS buffer (pH 10-11) were prepared. The enzyme activity at each pH was measured using these buffers at 30°C.

The results, as shown in Fig. 2-1, showed that the enzyme activity has a maximum at pH 8.0 in each of the two modified-type β-galactoside-α2,3-sialyltransferases (E319A and F318AE319A). The enzyme activity at each pH was a relative activity when the enzyme activity at pH 8.0 was defined to be 1. In contrast, the enzyme activity of wild-type N2C0 (not containing His × 6-Tag) not having modification has a maximum at pH 6.0.

### (2) Optimal temperature for enzyme activity of modified-type β-galactoside-α2,3-sialyltransferase (E319A and F318AE319A)

Enzyme activities were measured at a reaction temperature ranging from 10°C to 50°C at every 5°C using phosphate buffer (pH 6.0).

The results, as shown in Fig. 2-2, showed that the enzyme activity of wild-type N2C0 (ISH467-N2C0 not containing His × 6-Tag) not having variant has a maximum at 30°C. In contrast, the enzyme activity has a maximum at temperature of 35 to 40°C in each of the two modified-type β-galactoside-α2,3-sialyltransferases (E319A and F318AE319A).

### Example 6: Sialyltransferase activity and neuraminidase activity of each modified-type β-galactoside-α2,3-sialyltransferase

Example 2 revealed that mutation of the glutamic acid at position 319 of N2C0 to alanine inactivates the neuraminidase activity. Accordingly, in order to confirm the effect of replacement with an amino acid other than alanine, four modified-type β-galactoside-α2,3-sialyltransferases (E319V, E319S, E319K, and E319H) were produced, and crude enzyme solutions thereof were prepared by the procedure described below and subjected to measurement of sialyltransferase activity and neuraminidase activity.

A single colony of *E*. *coli* strain containing a ISH467-N2C0 variant produced in Example 1 (1) was inoculated in an LB medium (5 mL) containing an antibiotic, ampicillin (final concentration 100 µg/mL) and was pre-cultured at 30°C until the absorbance at 600 nm reached about 0.5, followed by addition of IPTG (isopropyl-β-D(-)-thiogalactopyranoside, manufactured by Wako Pure Chemical Industries, Ltd.) at a final concentration of 1mM. The cells were further shake-cultured at 30°C for 4 hours. The cells in 2 mL of the culture solution were collected by centrifugation. These cells were suspended in 200 µL of 20mM Bis-Tris buffer (pH 7.0) containing 0.3% Triton X-100 and 0.5M sodium chloride and sonicated under ice cooling. The resulting homogenate was subjected to measurement of activity as a crude enzyme solution. The sialyltransferase activity was measured by the method described in Example 2 (1), whereas the neuraminidase activity was measured by the method described in Example 4. The results thereof demonstrate that every modified-type β-galactoside-α2,3-sialyltransferase (E319V, E319S, E319K, and E319H) shows the sialyltransferase activity but not show the neuraminidase activity. The results are shown in Table 4.

**[Table 4]**

| Table 4 Enzyme activity of each modified-type-β-galactoside-α2,3-sialyltransferase | | | |
|---|---|---|---|
| | Enzyme activity (U/mg) | | |
| | Sialyltransferase | Neuraminidase | Reaction time* 1 |
| Ctrl *² | n.d. | n.d. | 108 h |
| WT | 0.15 | 0.11 | 2 h |
| E319A | 1.35 | n.d. | 108 h |
| E319H | 0.51 | n.d. | 108 h |
| E319K | 0.02 | n.d. | 108 h |
| E319S | 3.31 | n.d. | 108 h |
| E319V | 0.89 | n.d. | 108 h |

| | | | |
|---|---|---|---|
| n.d. Not detected *1 Neuraminidase redaction *2 Crude enzyme extract from E. coli transformed with vector only | | | |

### INDUSTRIAL APPLICABILITY

The modified-type N2C0 protein of the present invention is a protein having a β-galactoside-α2,3-sialyltransferase activity but substantially not having any neuraminidase activity. The enzyme of the present invention can reduce hydrolysis of sialic acid while maintaining the advantages of the enzyme derived from marine bacteria, i.e., a broad sugar acceptor substrate specificity in the sialyltransferase reaction. These properties of the protein of the present invention promises usefulness in synthesis of complex carbohydrate chains containing sialic acid.

### SEQUENCE LISTING FREE TEXT

SEQ ID NO: 1: nucleotide sequence encoding β-galactoside-α2,3-sialyltransferase N2C0 (prepared by removing the signal sequence from a β-galactoside-α2,3-sialyltransferase N0C0 gene derived from *Photobacterium phosphoreum* strain JT-ISH-467 and then adding Met to the N-terminus so as to allow recombinant expression)
SEQ ID NO: 2: amino acid sequence of β-galactoside-α2,3-sialyltransferase N2C0
SEQ ID NO: 3: nucleotide sequence encoding β-galactoside-α2,3-sialyltransferase N0C0 derived from *Photobacterium phosphoreum* strain JT-ISH-467
SEQ ID NO: 4: amino acid sequence of β-galactoside-α2,3-sialyltransferase N0C0
SEQ ID NO: 5: FLAG peptide
SEQ ID NO: 6: primer 467-23ST-N2-Nco
SEQ ID NO: 7: primer ISH467-23ST-C0-BamHI
SEQ ID NO: 8: primer ISH467-23ST-C0-His-BamHI
SEQ ID NO: 9: primer ISH467-23ST-F318A-F
SEQ ID NO: 10: primer ISH467-23ST-F318A-R
SEQ ID NO: 11: primer ISH467-23ST-E319A-F
SEQ ID NO: 12: primer ISH467-23ST-E319A-R
SEQ ID NO: 13: primer ISH467-23ST-F318AE319A-F
SEQ ID NO: 14: primer ISH467-23ST-F318AE319A-R
SEQ ID NO: 15: primer ISH467-23ST-S337A-F
SEQ ID NO: 16: primer ISH467-23ST-S337A-R
SEQ ID NO: 17: primer ISH467-23ST-E319V-F
SEQ ID NO: 18: primer ISH467-23ST-E319V-R
SEQ ID NO: 19: primer ISH467-23ST-E319S-F
SEQ ID NO: 20: primer ISH467-23ST-E319S-R
SEQ ID NO: 21: primer ISH467-23ST-E319K-F
SEQ ID NO: 22: primer ISH467-23ST-E319K-R
SEQ ID NO: 23: primer ISH467-23ST-E319H-F
SEQ ID NO: 24: primer ISH467-23ST-E319H-R
SEQ ID NO: 25: primer pTrc99A FW5
SEQ ID NO: 26: primer pTrc99A RV3

## Claims

1. A β-galactoside-α2,3-sialyltransferase protein wherein said protein comprises an amino acid sequence selected from the group consisting of:
(a) an amino acid sequence composed of 2nd to 386th amino acids of SEQ ID NO: 2 having replacement of the glutamic acid residue at position 319 of SEQ ID NO: 2 with an amino acid other than glutamic acid;
(b) an amino acid sequence having deletion, substitution, insertion, and/or addition of one or more amino acids in an amino acid sequence composed of 2nd to 386th amino acids of SEQ ID NO: 2, wherein at least the amino acid residue corresponding to the glutamic acid residue at position 319 of SEQ ID NO: 2 is replaced with an amino acid other than glutamic acid;
(c) an amino acid sequence having an identity of 80% or more with 2nd to 386th amino acids of SEQ ID NO: 2, wherein at least the amino acid residue corresponding to the glutamic acid residue at position 319 of SEQ ID NO: 2 is replaced with an amino acid other than glutamic acid; and
(d) an amino acid sequence encoded by a nucleotide sequence hybridizable with the complementary strand of 4th to 1158th nucleotides of SEQ ID NO: 1 under stringent conditions, wherein at least the codon corresponding to the 955th to the 957th nucleotides of SEQ ID NO: 1 is converted to a codon encoding an amino acid other than glutamic acid; and wherein said protein substantially does not have any neuraminidase activity and comprising.

2. The protein according to Claim 1, wherein the amino acid sequence further includes methionine, the 1st to 24th amino acids of SEQ ID NO: 4, or a signal peptide at the N-terminus of the amino acid sequence specified by any one of items (a) to (d) in Claim 1.

3. The protein according to Claim 1 or 2, wherein the glutamic acid residue at position 319 of SEQ ID NO: 2 or the amino acid residue corresponding to the glutamic acid residue is replaced with a neutral amino acid residue or a basic amino acid residue.

4. The protein according to Claim 1 or 2, wherein the glutamic acid residue at position 319 of SEQ ID NO: 2 or the amino acid residue corresponding to the glutamic acid residue is replaced with a neutral amino acid residue.

5. The protein according to any one of Claims 1 to 4, satisfying one or more requirements selected from the group consisting of the following items a) to 1):
a) the tyrosine residue at position 123 of SEQ ID NO: 2 or at a position corresponding thereto, is not modified or is replaced with tryptophan or phenylalanine;
b) the aspartic acid residue at position 124 of SEQ ID NO: 2 or at a position corresponding thereto, is not modified or is replaced with glutamic acid;
c) the aspartic acid residue at position 125 of SEQ ID NO: 2 or at a position corresponding thereto, is not modified or is replaced with glutamic acid;
d) the glycine residue at position 126 of SEQ ID NO: 2 or at a position corresponding thereto, is not modified or is replaced with alanine or valine;
e) the serine residue at position 127 of SEQ ID NO: 2 or at a position corresponding thereto,is not modified or is replaced with threonine;
f) the phenylalanine residue at position 291 of SEQ ID NO: 2 or at a position corresponding thereto, is not modified or is replaced with tryptophan or tyrosine;
g) the lysine residue at position 292 of SEQ ID NO: 2 or at a position corresponding thereto, is not modified or is replaced with arginine;
h) the glycine residue at position 293 of SEQ ID NO: 2 or at a position corresponding thereto, is not modified or is replaced with alanine or valine;
i) the histidine residue at position 294 of SEQ ID NO: 2 or at a position corresponding thereto, is not modified or is replaced with lysine or arginine;
j) the proline residue at position 295 of SEQ ID NO: 2 or at a position corresponding thereto, is not modified or is replaced with valine or isoleucine;
k) the serine residue at position 336 of SEQ ID NO: 2 or at a position corresponding thereto, is not modified or is replaced with threonine; and
l) the serine residue at position 337 of SEQ ID NO: 2 or at a position corresponding thereto, is not modified or is replaced with threonine.

6. A nucleic acid encoding a protein according to any one of Claims 1 to 5.

7. An isolated nucleic acid encoding a β-galactoside-α2,3-sialyltransferase protein, wherein said protein is a β-galactoside-α2,3-sialyltransferase protein substantially not having any neuraminidase activity, and the nucleic acid comprising a nucleotide sequence selected from the group consisting of:
(a) a nucleotide sequence composed of the 4th to 1158th nucleotides of SEQ ID NO: 1 having a codon encoding an amino acid other than glutamic acid in place of the codon of the 955th to the 957th nucleotides of SEQ ID NO: 1;
(b) a nucleotide sequence having deletion, substitution, insertion, and/or addition of one or more nucleotides in the 4th to 1158th nucleotides of SEQ ID NO: 1, wherein at least the codon corresponding to the 955th to the 957th nucleotides of SEQ ID NO: 1 is converted to a codon encoding an amino acid other than glutamic acid;
(c) a nucleotide sequence having an identity of 80% or more with the 4th to 1158th nucleotides of SEQ ID NO: 1, wherein at least the codon corresponding to the 955th to the 957th nucleotides of SEQ ID NO: 1 is converted to a codon encoding an amino acid other than glutamic acid; and
(d) a nucleotide sequence hybridizable with the complementary strand of the 4th to 1158th nucleotides of SEQ ID NO: 1 under stringent conditions, wherein at least the codon corresponding to the 955th to the 957th nucleotides of SEQ ID NO: 1 is converted to a codon encoding an amino acid other than glutamic acid.

8. An expression vector containing the nucleic acid according to Claim 6 or 7.

9. A method of producing a recombinant protein having a β-galactoside-α2,3-sialyltransferase activity, wherein said protein is a β-galactoside-α2,3-sialyltransferase protein substantially not having any neuraminidase activity, and the method comprises following steps:
1) transforming a host cell with an expression vector containing a nucleic acid according to Claim 6 or 7;
2) culturing the resulting transformed cell; and
3) isolating a protein having a β-galactoside-α2,3-sialyltransferase activity but substantially not having any neuraminidase activity from the cultured transformed cell or the culture supernatant thereof.
